# EUROPEAN PATENT APPLICATION

(11) **EP 4 734 667 A2**
(43) Date of publication of application: **29.04.2026**
(21) Application number: 26164652.5
(22) Date of filing: 29.06.2022
(51) Int. Cl.: H05G 1/02

(54) **IMAGE-GUIDED RADIATION THERAPY SYSTEMS AND METHODS THEREOF**

(62) Divisional of application: 22946036.5
(71) Applicant: Shanghai United Imaging Healthcare Co., Ltd., Shanghai 201807 (CN)
(72) Inventor: LIAO, Can, Shanghai, 201807 (CN); CAI, Bo, Shanghai, 201807 (CN); ZHANG, Wei, Shanghai, 201807 (CN)
(74) Representative: Wang, Bo

(57) **Abstract**

Embodiments of the present disclosure provide an image-guided radiation therapy system and a method thereof. The system includes a first detection assembly, at least one imaging source, and a radiation source. The first detection assembly may be configured to determine a first image of a target region of an object by receiving a photon signal generated by a radioactive isotope within the object. The at least one imaging source may be configured to determine a second image by emitting an imaging beam to the target region of the object. The radiation source may be configured to emit a therapeutic beam to the target region of the object. The first detection assembly and the at least one imaging source may be mounted on a first rotatable part, the radiation source may be mounted on a second rotatable part, and the first rotatable part and the second rotatable part may be coaxially and coplanarly arranged. The first rotatable part may be configured to be rotatable independently relative to the second rotatable part.

## Description

### TECHNICAL FIELD

The present disclosure relates to the field of medical devices, and in particular to an image-guided radiation therapy system and a method thereof.

### BACKGROUND

Radiation therapy is a localized treatment for a specific target tissue (e.g., a malignant tumor), where before, during, or after the treatment, dose and geometric data are examined to ensure the correct positioning of a patient and to verify that administered radiation is in alignment with a previously planned treatment. This process is called image-guided radiation therapy, which involves using an imaging system to monitor the target tissue while radiation therapy reaches the target tissue.

### SUMMARY

The present invention is defined by the appended claims.

### BRIEF DESCRIPTION OF THE DRAWINGS

The present disclosure is further illustrated by way of exemplary embodiments, which are described in detail through the accompanying drawings. These embodiments are not limiting, and in these embodiments, the same numbering indicates the same structure, wherein:
FIG. 1 is a schematic diagram illustrating an exemplary scenario of an application of an image-guided radiation therapy system according to some embodiments of the present disclosure;
FIGs. 2(a) and 2(b) are schematic diagrams illustrating structures of an exemplary image-guided radiation therapy system according to some embodiments of the present disclosure;
FIG. 3 is a schematic diagram illustrating a structure of an exemplary image-guided radiation therapy system according to some embodiments of the present disclosure;
FIG. 4 is a schematic diagram illustrating a structure of an exemplary image-guided radiation therapy system according to some embodiments of the present disclosure;
FIG. 5 is a schematic diagram illustrating a structure of an exemplary image-guided radiation therapy system according to some embodiments of the present disclosure;
FIGs. 6(a) and 6(b) are schematic diagrams illustrating structures of an exemplary image-guided radiation therapy system according to some other embodiments of the present disclosure;
FIG. 7 (a) and 7 (b) are schematic diagrams illustrating side views of an exemplary image-guided radiation therapy system according to some embodiments of the present disclosure;
FIG. 8 is a schematic diagram illustrating hardware and/or software components of an exemplary computing device according to some embodiments of the present disclosure;
FIG. 9 is a schematic diagram illustrating hardware and/or software components of an exemplary terminal device according to some embodiments of the present disclosure;
FIG. 10 is a modular schematic diagram illustrating an exemplary image-guided radiation therapy system according to some embodiments of the present disclosure;
FIG. 11 is a flowchart illustrating an exemplary process of an image-guided radiation therapy method according to some embodiments of the present disclosure;
FIG. 12 is a flowchart illustrating another exemplary process of an image-guided radiation therapy method according to some embodiments of the present disclosure; and
FIG. 13 is a flowchart illustrating another exemplary process of an image-guided radiation therapy method according to some embodiments of the present disclosure.

### DETAILED DESCRIPTION

In order to more clearly illustrate the technical solutions of the embodiments of the present disclosure, the accompanying drawings for the description of the embodiments will be described below. Obviously, the accompanying drawings in the following description are only some examples or embodiments of the present disclosure, and it is possible for a person of ordinary skill in the art to apply the present disclosure to other similar scenarios in accordance with these accompanying drawings without creative labor. Unless obviously obtained from the context or the context illustrates otherwise, the same numeral in the drawings refers to the same structure or operation.

It should be understood that the terms "system," "device," "unit," and/or "module" are used herein as a way to distinguish between different components, elements, parts, sections, or assemblies at different levels. However, if other words may achieve the same purpose, the terms may be replaced with alternative expressions.

As indicated in the present disclosure and the claims, unless the context clearly suggests an exception, the words "one," "a," "a kind of," and/or "the" do not refer specifically to the singular but may also include the plural. In general, the terms "include" and "comprise" suggest only the inclusion of clearly identified steps and elements, which do not constitute an exclusive list, and the method or device may also include other steps or elements.

The present disclosure uses flowcharts to illustrate the operations performed by the system according to some embodiments of the present disclosure. It should be understood that the operations described herein are not necessarily executed in a specific order. Instead, they may be executed in reverse order or simultaneously. Additionally, other operations may be added to these processes or certain steps may be removed.

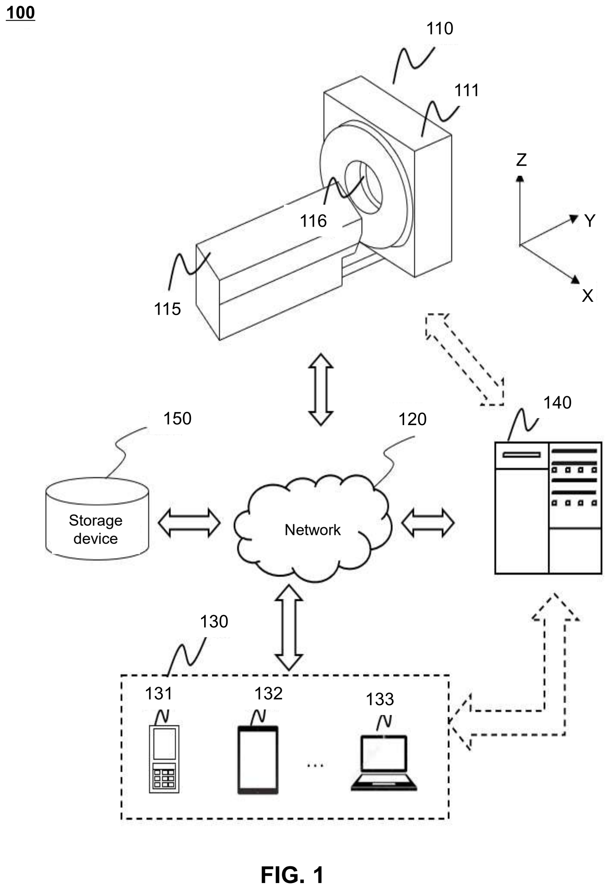

FIG. 1 is a schematic diagram illustrating an exemplary scenario of an application of an image-guided radiation therapy system according to some embodiments of the present disclosure. In some embodiments, as shown in FIG. 1, the image-guided radiation therapy system 100 may include a medical device 110, a network 120, at least one terminal device 130, a processing device 140, and a storage device 150. In some embodiments, various components of the image-guided radiation therapy system 100 may be interconnected through the network 120. For example, the medical device 110 and the at least one terminal device 130 may connect or communicate through the network 120. In some embodiments, the image-guided radiation therapy system 100 may be used to provide radiation therapy (e.g., stereotactic radiation surgery and/or precise radiation therapy) to a target region (e.g., a lesion, a tumor, etc.) of a patient as well as any other radiation therapy locations. In some embodiments, the image-guided radiation therapy system 100 may include a treatment planning system, an image-guided radiation therapy system, or the like.

In some embodiments, the image-guided radiation therapy system 100 may perform image-guided radiation therapy. For example, the image-guided radiation therapy system 100 may use X-ray imaging to monitor the target region (e.g., the lesion, the tumor, etc.) to be treated within an object (e.g., the patient). In this case, the medical device 110 may include a treatment device (also referred to as a treatment assembly) and an imaging device (also referred to as an imaging assembly). The treatment device may be used to perform radiation therapy on the target area with a therapeutic beam. The imaging device (e.g., a first detection assembly, a computed tomography (CT) imaging assembly) may be used to image the target region and/or a surrounding normal tissue before, during, or after radiation therapy (e.g., two-dimensional (2D) imaging, three-dimensional (3D) imaging, or four-dimensional (4D) imaging). In this way, an anatomical structure of the target region (e.g., a targeted region, an organ at risk, etc.) and a movement or deformation thereof may be detected, and a position of the patient and/or the therapeutic beam may be adjusted to more accurately perform radiation therapy upon the target region.

In some embodiments, the treatment device (the treatment assembly) may include a treatment head. The treatment head may be used to emit the therapeutic beam to the object to perform radiation therapy on the target region inside the object. In some embodiments, the treatment head may include an upper treatment head and a lower treatment head.

The upper treatment head may include a radiation source for emitting the therapeutic beam. In some embodiments, the upper treatment head may include at least one of a microwave part, an acceleration part, or a cooling part. The microwave part may generate an electromagnetic field for accelerating an electron beam to generate a high-energy electron beam. The acceleration part (e.g., an acceleration tube) may be used to accelerate the electron beam. The cooling part may be used to cool the parts of the upper treatment head, such as the microwave part, the acceleration part, etc.

The lower treatment head may include a collimation part used to restrict the radiation range of the therapeutic beam emitted by the radiation source. In some embodiments, the lower treatment head may include at least one of a beam modifier filter, an ionization chamber, a gantry, a multi-leaf collimator (MLC), etc. The beam modifier filter may be used to adjust the energy distribution of the therapeutic beam. The ionization chamber may ionize gas inside the ionization chamber to detect the parameters (e.g., intensity, flatness, and symmetry) of the therapeutic beam. The radiation range of the therapeutic beam (or filtered therapeutic beam) may be adjusted by adjusting the position of the gantry and/or leaves of the multi-leaf collimator (MLC).

In some embodiments, the imaging device may include at least one imaging source (e.g., a CT tube, a digital radiography (DR) tube) and/or a detector (e.g., the first detection assembly, a CT detector, a DR detector, etc.). The imaging source may emit an imaging beam toward the target region of the object. The detector may receive at least a portion of the imaging beam to image the object (e.g., generate a second image). In some embodiments, the detector (e.g., a positron emission tomography (PET) detector, a single photon emission computed tomography (SPECT) detector) may be used to receive a photon signal generated by a radioactive isotope within the object for imaging (e.g., generate a first image of the target region of the object).

In some embodiments, the imaging device may include one of an X-ray device, a digital radiography (DR) imaging device, a direct-digit radiography (DDR) imaging device, a computed tomography (CT) imaging device, three-dimensional (3D) CT, four-dimensional (4D) CT, an ultrasound imaging assembly, a fluoroscopy imaging assembly, a magnetic resonance imaging (MRI) device, a single-photon emission computed tomography (SPECT) device, a positron emission computed tomography (PET) device, or a combination thereof. The provided examples of imaging devices are for illustrative purposes only and do not intend to limit the scope of the present disclosure.

In the present disclosure, an x-axis, a y-axis, and a z-axis as shown in FIG. 1 form an orthogonal coordinate system. The x-axis and the y-axis shown in FIG. 1 are horizontal, while the z-axis is vertical. As shown in FIG. 1, when viewed from a front side to face the medical device 110, a positive x-direction along the x-axis is from a left to a right of the medical device 110; a positive z-direction along the z-axis is from a bottom to a top of the medical device 110; and a positive y-direction along the y-axis is the direction in which the object is moved out of a bore 116 of the medical device 110.

In some embodiments, the medical device 110 may include a frame 111 and a bed 115. The frame 111 may be configured to support the treatment device and the imaging device. The frame 111 may rotate around the object (e.g., the patient) positioned within a radiation field (e.g., a region covered by a radiation beam emitted by the treatment head and/or the imaging assembly) of the medical device 110. In some embodiments, the bed 115 may be configured to support the object. The bed 115 may include a plurality of degrees of freedom, such as, translational degrees of freedom along three coordinate directions (i.e., an x-axis direction, a y- axis direction, and a z-axis direction as shown in FIG. 1) and/or rotational degrees of freedom around the three coordinate directions. Therefore, the bed 115 may move the object along any direction in the 3D coordinate system shown in FIG. 1. Merely as an illustrative example, the bed 115 may move the object along the y-axis direction shown in FIG. 1 into or out of the radiation field of the medical device 110.

In some embodiments, the object may include a biological or a non-biological object. For example, the object may include a patient, an artificial object, etc. As another example, the object may include a phantom. As yet another example, the object may include a specific part, an organ, and/or a tissue of the patient. For example, the object may include the head, the brain, the neck, the body, a shoulder, an arm, the chest, the heart, the stomach, a blood vessel, a soft tissue, a knee, a foot, etc., or any combination thereof.

The network 120 may include any suitable network capable of facilitating information and/or data exchange for the image-guided radiation therapy system 100. In some embodiments, at least one component (e.g., the medical device 110, the at least one terminal device 130, the processing device 140, the storage device 150) of the image-guided radiation therapy system 100 may exchange information and/or data with at least one other component of the image-guided radiation therapy system 100 through the network 120. For example, the processing device 140 may obtain an image (e.g., the first image, the second image) of an imaged subject (e.g., the object) from the medical device 110 through the network 120. The network 120 may include a public network (e.g., the Internet), a private network (e.g., a local area network (LAN)), a wired network, a wireless network (e.g., a 802.11 network, a Wi-Fi network), a frame relay network, a virtual private network (VPN), a satellite network, a telephone network, a router, a hub, a switch, a server computer, and/or any combination thereof. In some embodiments, the network 120 may include at least one network access point. For example, the network 120 may include a wired and/or a wireless network access point, such as a base station and/or an internet exchange point, and at least one component of the image-guided radiation therapy system 100 may connect to the network 120 through the access point to exchange data and/or information.

The at least one terminal device 130 may communicate and/or connect with the medical device 110, the processing device 140, and/or the storage device 150. For example, an operator may adjust a current acquisition parameter of the medical device 110 through the at least one terminal device 130. As another example, the operator may input a scan protocol through the at least one terminal device 130, which is then stored by the processing device 140 in the storage device 150. As yet another example, the acquisition parameter determined by the processing device 140 may be displayed on the at least one terminal device 130. In some embodiments, the at least one terminal device 130 may include a mobile device 131, a tablet computer 132, a laptop computer 133, etc., or any combination thereof. For example, the mobile device 131 may include a mobile control handle, a personal digital assistant (PDA), a smartphone, etc., or any combination thereof.

In some embodiments, the at least one terminal device 130 may include an input device, an output device, etc. The input device may include keyboard input, touch screen (e.g., with tactile or haptic feedback) input, voice input, eye tracking input, gesture tracking input, brain monitoring system input, image input, video input, or any other similar input mechanisms. Input information received by the input device may be transmitted to the processing device 140 via a bus for further processing. Other types of input devices may include cursor control devices such as a mouse, a trackball, or cursor direction keys. In some embodiments, the operator (e.g., a technician or a doctor) may input the scan protocol through the input device. The output device may include a display, a speaker, a printer, or any combination thereof. The output device may be used to output the acquisition parameter determined by the processing device 140, etc. In some embodiments, the at least one terminal device 130 may be part of the processing device 140.

The processing device 140 may process data and/or information obtained from the medical device 110, the at least one terminal device 130, the storage device 150, or other components of the image-guided radiation therapy system 100. For example, the processing device 140 may generate the first image of the target region of the object by making the first detection assembly of the medical device 110 receive a photon signal generated by a radioactive isotope within the object. As another example, the processing device 140 may generate the second image of the target region of the object by guiding the at least one imaging source of the medical device 110 to emit an imaging beam towards the object. In some embodiments, the processing device 140 may be a single server or a server group. The server group may be centralized or distributed. In some embodiments, the processing device 140 may be local or remote. For example, the processing device 140 may access information and/or data from the medical device 110, the storage device 150, and/or the at least one terminal device 130 through the network 120. As another example, the processing device 140 may directly connect to the medical device 110, the at least one terminal device 130, and/or the storage device 150 to access information and/or data. In some embodiments, the processing device 140 may be implemented on a cloud platform. For example, the cloud platform may include a private cloud, a public cloud, a hybrid cloud, a community cloud, a distributed cloud, an inter-cloud, a multi-cloud, or any combination thereof.

The storage device 150 may store data, one or more instructions, and/or any other information. In some embodiments, the storage device 150 may store data obtained from the medical device 110, the at least one terminal device 130, and/or the processing device 140. In some embodiments, the storage device 150 may store data and/or an instruction used by the processing device 140 to execute or implement an exemplary method described in the present disclosure. In some embodiments, the storage device 150 may include a high-capacity storage, a removable storage, a volatile read-write storage, a read-only memory (ROM), etc., or any combination thereof. In some embodiments, the storage device 150 may be implemented on the cloud platform.

In some embodiments, the storage device 150 may connect to the network 120 to communicate with at least one other component in the image-guided radiation therapy system 100 (e.g., the at least one terminal device 130, the processing device 140). At least one component in the image-guided radiation therapy system 100 may access data stored in the storage device 150 through the network 120. In some embodiments, the storage device 150 may be part of the processing device 140.

It should be noted that the above description is provided for illustrative purposes only and is not intended to limit the scope of the present disclosure. Various changes and modifications may be made by those skilled in the art under the guidance of the content of the present disclosure. The features, structures, methods, and other features of the exemplary embodiments described in the present disclosure may be combined in various ways to obtain additional and/or alternative exemplary embodiments. For example, the storage device 150 may be a data storage device including a cloud computing platform. However, these changes and modifications do not depart from the scope of the present disclosure.

During a radiation therapy cycle, an anatomy and/or a tumor of the patient may continuously change over time. Therefore, in addition to imaging of an anatomical structure of the patient, functional imaging, molecular metabolic imaging, etc., are also needed during the radiation therapy to observe changes in the tumor of the patient, providing the doctor with more information for clinical decision-making. In some embodiments, a type of image to be obtained may be selected based on a clinical need. In some embodiments, treatment planning may be carried out and/or adjusted based on a medical image obtained from the imaging device before each radiation therapy session. In some embodiments, the medical image may also be used for real-time monitoring and tracking of the tumor.

Exemplary embodiments of the present disclosure provide the image-guided radiation therapy system. The image-guided radiation therapy system may include the first detection assembly, the at least one imaging source, and the radiation source. The first detection assembly and the at least one imaging source may be mounted on a first rotatable part for imaging the object (e.g., determining the first image or the second image). The radiation source (e.g., the upper treatment head) may be mounted on a second rotatable part for emitting a therapeutic beam towards the target region of the object. In some embodiments, the first rotatable part and the second rotatable part are configured to be able to rotate independently of each other. In some embodiments, the first rotatable part and the second rotatable part may be coaxially and coplanarly arranged. In some embodiments, the first detection assembly or the at least one imaging source may be configured to be movable relative to the first rotatable part. In some embodiments, the first detection assembly may move, relative to the first rotatable part, between a first position and a second position. In some embodiments, the at least one imaging source may move between the third position and the fourth position relative to the first rotatable part.

In some embodiments, the medical device 110 may include the first detection assembly and the radiation source. The first detection assembly may be mounted on the first rotatable part, and the radiation source may be mounted on the second rotatable part. In some embodiments, the first detection assembly may include a PET detector or a SPECT detector. In some embodiments, the first detection assembly may include an arc-shaped detector. In some embodiments, detectors other than the PET detector and the SPECT detector (e.g., a therapeutic beam detector, a CT detector, a DR detector) may be referred to as a second detection assembly. In some embodiments, the second detection assembly may include an arc-shaped detector and/or a flat panel detector.

In some embodiments, the first rotatable part may connect to a frame (e.g., the frame 111), and the first detection assembly may connect to the first rotatable part. In some embodiments, the first detection assembly may generate the first image of the target region of the object by receiving a photon signal generated by a radioactive isotope within the object. For example, a substance (e.g., glucose, protein, nucleic acid, fatty acid, etc.) essential to biological metabolism may be labeled with a short-lived radioactive isotope (e.g., ¹⁸F, ¹¹C, etc.), injected into the object's body, and the first detection assembly may generate the first image of the target region of the object by receiving the photon signal generated by decay of the injected substance in the object.

In some embodiments, the first detection assembly may be configured to be movable relative to the first rotatable part. In some embodiments, the first detection assembly may move, relative to the first rotatable part, between the first position and the second position.

In some embodiments, the first detection assembly may be movable relative to the first rotatable part through a movable structure. In some embodiments, the first rotatable part may include a guide rail, and the first detection assembly may move between the first position and the second position along the guide rail. In some embodiments, the first detection assembly may include a motion part that matches the guide rail and a corresponding driving mechanism. The first detection assembly may switch between the first position and the second position by driving the motion part along the guide rail through the driving mechanism. For example, the first rotatable part along the y-axis direction (as shown in FIG. 1) may be provided with a linear guide rail, and the first detection assembly may be provided with a motion part (e.g., a slider, a ball, a roller, a needle, etc.) that matches the linear guide rail and a corresponding driving mechanism. The driving mechanism may drive the motion part on the first detection assembly to move along the linear guide rail by a preset distance, allowing the first detection assembly to switch between the first position and the second position. In some embodiments, the guide rail may include but is not limited to a flat guide rail, a cylindrical guide rail, a dovetail guide rail, a V-shaped guide rail, etc., or a combination thereof.

In some embodiments, the first detection assembly may move relative to the first rotatable part through other movable structures, details of which are not limited in the present disclosure. For example, the first rotatable part may be provided with a guiding shaft, and the first detection assembly may be provided with a linear bearing that matches the guiding shaft. The first detection assembly may slide connect with the first rotatable part through the linear bearing and the guiding shaft, allowing the first detection assembly to switch between the first position and the second position. As another example, the first detection assembly may be mounted on the first rotatable part through a cam mechanism, a linear motor, or other structures, enabling switching of the first detection assembly between the first position and the second position. As yet another example, the first detection assembly may be mounted on the first rotatable part through a magnetic levitation sliding structure, thereby achieving movement of the first detection assembly between the first position and the second position relative to the first rotatable part.

In some embodiments, the first detection assembly may switch between the first position and the second position by moving the preset distance. In some embodiments, a moving distance (e.g., the preset distance) of the first detection assembly on the first rotatable part may be determined based on a thickness of the first detection assembly. For example, if a thickness of the PET detector along an axial direction of the first rotatable part is 30 cm, a distance between the first position and the second position may be 20 cm or 25 cm (i.e., the first detection assembly moves linearly by 20 cm or 25 cm to switch from the first position to the second position or vice versa). In some embodiments, the moving distance of the first detection assembly may be determined based on another imaging assembly. For example, when the CT imaging assembly is mounted on the first rotatable part, the distance between the first position and the second position may be determined based on an emission direction and a beam range of an imaging beam during CT imaging (e.g., the second position of the first detection assembly needs to allow at least a portion of the imaging beam to irradiate the object or the target region thereof). As used herein, the term "thickness" refers to a length of the first detection assembly along the axial direction (e.g., the y-axis direction in FIG. 1) of the first rotatable part or the frame (e.g., the frame 111).

In some embodiments, the first detection assembly may move relative to the first rotatable part through linear translational motion. In some embodiments, the first detection assembly may move linearly along a rotational axis direction of the first rotatable part. For example, the first detection assembly may move back and forth the preset distance along the rotational axis direction (e.g., the y-axis direction shown in FIG. 1) of the frame 111 of the medical device 110, achieving movement between the first position and the second position. In some embodiments, the first detection assembly may move relative to the first rotatable part through rotational motion. For example, the PET detector may move out or back into an isocenter plane along the rotational axis of the frame 111 of the medical device 110 by rotating clockwise or counterclockwise, achieving movement between the first position and the second position. Similarly, the SPECT detector may rotate along the rotational axis of the frame of the medical device 110, rotating by a specific angle to move between the first position and the second position.

In some embodiments, the first detection assembly may include at least two sets of detectors. Each set of detectors may include at least one detector. For example, the first detection assembly may include two sets of PET/SPECT detectors or three sets of PET/SPECT detectors. In some embodiments, at least one detector among the at least two sets of detectors may move relative to the first rotatable part. For example, one set of detectors among the at least two sets of detectors, which are adjacent to the at least one imaging source, may move, relative to the first rotatable part, between the first position and the second position. As another example, the at least two sets of detectors may simultaneously move, relative to the first rotatable part, between the first position and the second position. As yet another example, each detector among the at least two sets of detectors may respectively move, relative to the first rotatable part, between the first position and the second position.

In some embodiments, the second rotatable part may connect to the frame (e.g., the frame 111), and the radiation source may connect to the second rotatable part. The second rotatable part may be configured to make the radiation source (e.g., the upper treatment head) rotate, emitting the therapeutic beam towards the target region of the object for treatment. In some embodiments, the second rotatable part may drive the radiation source to rotate by rotating along a central axis of the frame. In some embodiments, the target region refers to a targeted region (e.g., a tumor region) of the object that requires radiation therapy. In some embodiments, the radiation source of the therapeutic beam may include at least one of an alpha(α)-ray source, a beta(β)-ray source, a gamma(γ)-ray source, or the like.

In some embodiments, the medical device 110 may include a fixed portion (e.g., a stator), and the first rotatable part and the second rotatable part may connect to the fixed portion. The fixed portion may be configured to support the emission of the therapeutic beam from the radiation source and support the imaging device (e.g., the first detection assembly, the CT imaging assembly, a DR imaging assembly) to receive the photon signal or emit the imaging beam. In some embodiments, the first rotatable part, the second rotatable part, and the fixed portion may be integrated into the frame (e.g., an O-shaped frame). In some embodiments, the first rotatable part and/or the second rotatable part may have an irregular shape, such as a circular or polygonal structure or other structures capable of rotating relative to a center, details of which are not limited in the present disclosure do not limit this.

In some embodiments, the first rotatable part and the second rotatable part may connect to the fixed portion through a first bearing and a second bearing, respectively. In some embodiments, the first rotatable part may connect to the fixed portion through the first bearing, and the second rotatable part may connect to the first rotatable part through the second bearing. In this case, it is necessary to ensure that when the first rotatable part rotates independently, the second rotatable part may rotate synchronously with the first rotatable part or maintain a certain distance of relative movement (i.e., when the first rotatable part rotates, the relative position between the second rotatable part and the first rotatable part remains unchanged), thereby avoiding deviation of rotation axes (e.g., a center of the rotation plane where the first rotatable part is located and a center of the rotation plane where the second rotatable part is located) the first rotatable part and the second rotatable part and/or a change in a distance between the first rotatable part and the second rotatable part.

In some embodiments, the first rotatable part may rotate on a first rotation plane (also referred to as an imaging plane), and the second rotatable part may rotate on a second rotation plane (also referred to as a treatment plane). The first rotation plane and the second rotation plane are different. In some embodiments, the first rotation plane and the second rotation plane are perpendicular to a rotational axis (e.g., the y-axis direction shown in FIG. 1) of the frame (e.g., the frame 111) and pass through a center (e.g., a center of the radiation therapy device, a center of the imaging device) of the medical device 110. In some embodiments, the first rotatable part and the second rotatable part may be coaxially arranged.

In some embodiments, the imaging assembly and the treatment assembly may be isocentrically configured. For example, the imaging assembly and the treatment assembly may be made isocentric by coinciding a central axis of the first rotatable part and a central axis of the second rotatable part. As used herein, isocentric refers to that a mechanical center (e.g., a center of the frame, a center of the first rotatable part, a center of the second rotatable part) of the medical device 110 coincides or closely coincides with a center of a radiation therapy region or an imaging region.

In some embodiments, the first detection assembly and the radiation source may be coplanar. In some embodiments, the first detection assembly and the radiation source may be coplanar in a longitudinal direction of the first rotatable part or the second rotatable part. For example, the first rotation plane and the second rotation plane may be made coplanar in the longitudinal direction (in a same plane perpendicular to the y-axis direction shown in FIG. 1), achieving the longitudinal coplanarity of the first detection assembly and the radiation source. In some embodiments, the first detection assembly may be coplanar with the radiation source (e.g., the upper treatment head) when located at the first position or the second position. In some embodiments, the first detection assembly may be partially coplanar or non-coplanar with the radiation source (e.g., the upper treatment head) when located at the first position or the second position. For example, when the second position is a position where the PET detector is partially moved out of the isocentric plane along an isocenter (e.g., a y-axis negative direction shown in FIG. 1) of the medical device 110, i.e., when the PET detector intersects with the isocentric plane, then the PET detector is coplanar with the upper treatment head portion. As another example, when the second position is a position where the PET detector is completely moved out of the isocentric plane along the isocenter (e.g., the y-axis negative direction shown in FIG. 1) of the medical device 110, i.e., when the PET detector is away from or tangent to the isocentric plane, at this time the PET detector is non-coplanar with the upper treatment head. In some embodiments, the isocentric plane may correspond to a coplanar plane (e.g., a longitudinal (vertical direction) plane corresponding to an entrance of the bore 116 in FIG. 1) of the first rotatable part and the second rotatable part.

In some embodiments, the medical device 110 may further include at least one imaging source. The at least one imaging source is mounted on the first rotatable part and is isocentric with the first detection assembly and the radiation source. In some embodiments, when the first detection assembly is at the first position or the second position, the at least one imaging source, the first detection assembly, and the radiation source are coplanar in a longitudinal direction. In some embodiments, the first rotatable part may drive the at least one imaging source and/or the first detection assembly to rotate, thereby determining the first image and/or the second image of the target object. In some embodiments, the first rotatable part may drive the at least one imaging source and/or the first detection assembly to rotate at a higher speed (e.g., 0.1 sec/rotation-1 sec/rotation, such as 0.25 sec/rotation), thereby improving imaging speed. In addition, driving the at least one imaging source and/or the first detection assembly to rotate at a higher speed may reduce imaging artifacts and improve imaging quality.

In some embodiments, the at least one imaging source may be configured to emit an imaging beam toward the object to generate the second image of the target region of the object. In some embodiments, the at least one imaging source may include a computed tomography (CT) imaging source, a digital radiography (DR) imaging source, and/or an X-ray imaging source, and the present disclosure does not limit this.

In some embodiments, the medical device 110 may also include other imaging assemblies. For example, the medical device 110 may include a CT/DR detector, a high-voltage part, a cooling part, etc., or any combination thereof. The CT/DR detector may detect the imaging beam emitted by the CT imaging source/DR imaging source to generate the second image of the object. In some embodiments, the CT/DR detector may be placed to face the CT imaging source/DR imaging source. The high-voltage part may be used to accelerate the electron beam inside the imaging source (e.g., a tube) to generate the imaging beam. The cooling part may be used to cool components of the imaging device, such as the high-voltage part, the tube, etc.

In some embodiments, the first detection assembly may include at least three sets of detectors. In some embodiments, the at least one imaging source may be mounted between at least two sets of detectors among the at least three sets of detectors. For example, in three sets of detectors, a first set of detectors may be located on one side of the upper treatment head, and a second set of detectors and a third set of detectors may be located on another side of the upper treatment head and opposite to the first set of detectors, and the CT tube may be located between the second set of detectors and the third set of detectors. Wherein, "one side" and "another side" of the upper treatment head refer to two sides of the upper treatment head along a radial direction of the frame (e.g., the z-axis direction shown in FIG. 1).

In some embodiments, the first detection assembly may include at least two sets of detectors. In some embodiments, the at least two sets of detectors may be placed to face each other. In some embodiments, when the at least two sets of detectors are in the first position, the at least one imaging source may be configured to be obscured by at least one detector among the at least two sets of detectors. For example, the first set of detectors may be located on one side of the upper treatment head, the second set of detectors may be located on another side of the upper treatment head and opposite to the first set of detectors, and the CT tube may be on either side of the upper treatment head and obscured by the first or the second set of detectors.

In some embodiments, the at least one imaging source and/or a corresponding imaging assembly (e.g., the CT detector, the DR detector) may be configured to be movable, relative to the first rotatable part, between the third position and the fourth position. In some embodiments, the at least one imaging source and/or the corresponding imaging assembly may be movable, relative to the first rotatable part, between the third position and the fourth position via a movable structure. In some embodiments, the imaging assembly may be configured to move, relative to the first rotatable part, between the third position and the fourth position. For example, the CT tube and the CT detector arranged opposite to the CT tube may rotate about a rotation axis of the first rotatable part to achieve movement between the third position and the fourth position.

In some embodiments, when the at least one imaging source is in the third position, at least one detector among the at least two sets of detectors of the first detection assembly may be obscured by the at least one imaging source. For example, the first set of detectors may be on one side of the upper treatment head, the second set of detectors may be on another side of the upper treatment head and opposite to the first set of detectors, and the CT tube may be on either side of the upper treatment head and obscured by the first or the second set of detectors.

In some embodiments, the radiation source, the at least one imaging source, and the first detection assembly may be located on the same plane (e.g., the isocentric plane). In some embodiments, the collimation part and a detector (also referred to as a therapeutic beam detector) corresponding to the radiation source may be located on this plane. In some embodiments, the collimation part and the detector corresponding to the radiation source may be movable relative to the second rotatable part. For example, the detector corresponding to the radiation source may be movable translationally along a central axis direction of the second rotatable part or rotate about a rotation axis of the second rotatable part to avoid obscuring a detection range of the first detection assembly. In some embodiments, an imaging detector (e.g., the CT detector, the first detection assembly) may detect a kilovoltage (kV) radiation beam and/or a megavoltage (MV) radiation beam, and the therapeutic beam detector (e.g., an electronic portal imaging device (EPID)) may detect the kilovoltage (kV) radiation beam and/or the megavoltage (MV) radiation beam. In some embodiments, the therapeutic beam detector may be movable relative to the first rotatable part. For example, the therapeutic beam detector may rotate about the rotation axis of the first rotatable part or move translationally along a central axis direction of the first rotatable part.

Due to the independent movement of the first rotatable part relative to the second rotatable part, the rotation of the imaging assembly can be unaffected by the second rotatable part (e.g., a mass or a rotation speed of the second rotatable part). The first detection assembly is configured to be movable, relative to the first rotatable part, between the first position and the second position, ensuring that the CT/DR imaging is not affected by the first detection assembly, thus guaranteeing imaging efficiency and image quality of second imaging (e.g., CT imaging and/or DR imaging). The radiation source or the at least one imaging source is configured to be movable relative to the first rotatable part, ensuring that PET imaging is not affected by the radiation source and/or the at least one imaging source, thus guaranteeing the image quality and imaging efficiency of the first image.

In some embodiments, the imaging beam may cover an imaging region, and the therapeutic beam may cover a treatment region. The at least one imaging source (e.g., the CT tube) and the radiation source (e.g., the upper treatment head) may be configured such that the treatment region and the imaging region at least partially overlap and/or the treatment region and the imaging region are isocentric. In some embodiments, the target region (e.g., a region to be treated) of the object may be placed in an overlapping region between the treatment region and the imaging region. In some embodiments, an isocenter of the radiation therapy device (e.g., the treatment assembly) may coincide with an isocenter of a computed tomography scanning device (e.g., the CT imaging assembly), and the target region of the object may be placed at the coincident isocenter. As used herein, "coincident" refers to that a deviation between the isocenter of the radiation therapy device and the isocenter of the computed tomography scanning device is within a preset range.

In some embodiments, because the first rotatable part may rotate at a high speed, a thickness of the detector (e.g., the therapeutic beam detector, the PET detector, the CT detector, the DR detector) in the first rotatable part does not affect the image quality. In some embodiments, the thickness of the detector (e.g., the therapeutic beam detector, the PET detector, the CT detector, the DR detector) in the first rotatable part may be less than a threshold (e.g., 3 cm, 4 cm, 5 cm, 7 cm, 8 cm, etc.). For example, the CT detector or the PET detector may include an arc-shaped detector, and the DR detector may be a flat-panel detector. In this case, the thickness along this axis of the frame may be reduced, thus reducing the spatial requirements for placing the medical device 110.

In some embodiments, the medical device 110 may also include a control part. In some embodiments, the control part may be used to control the first rotatable part and the second rotatable part to remain stationary, rotate synchronously, or rotate independently. For example, the first rotatable part and the second rotatable part may receive a control signal from the control part to achieve synchronous or independent rotation. As another example, the control signal may control the first rotatable part and the second rotatable part to rotate at the same speed and in the same direction. In some embodiments, the control part may be used to control the first detection assembly, the at least one imaging source and/or the corresponding imaging assembly, and the therapeutic beam detector to move relative to the first rotatable part. In some embodiments, a count of the control parts may be one or more. For example, the count of the control parts is one, and the one control part is used to control the first rotatable part and the second rotatable part to rotate. As another example, the count of the control parts may be two. The two control parts can be respectively used to control the first rotatable part and the second rotatable part to rotate. The two control parts may be further used to control the first detection assembly to move between the first position and the second position, or control the at least one imaging source to move between the third position and the fourth position.

In some embodiments, the medical device 110 may also include a locking part. The locking part may be used to lock the first rotatable part and the second rotatable part, such that the first rotatable part and the second rotatable part may rotate synchronously. In some embodiments, the locking part may align the collimation part (e.g., the lower treatment head) on the first rotatable part with the radiation source (e.g., the upper treatment head) on the second rotatable part by locking the first and the second rotatable parts. In some embodiments, before radiation therapy, the first rotatable part and the second rotatable part may be in an unlocked state, and independent rotation of the first rotatable part (e.g., rotation of the first rotatable part, stationary second rotatable part) may be used to image (e.g., generating the first image, the second image) a region of interest of the object (e.g., the target region to be treated). In this case, the first rotatable part is not affected by the second rotatable part and may rotate at a high speed (e.g., 0.1 sec/rotation - 1 sec/rotation), which could allow fast completion of the imaging process. In some embodiments, before radiation therapy, the first rotatable part and the second rotatable part may be synchronously rotated (e.g., the first rotatable part and the second rotatable part rotate at a same speed in a same direction), i.e., the first rotatable part and the second rotatable part are relatively stationary, to image (e.g., generating the first image or the second image) the region of interest of the object (e.g., the target region to be treated).

In some embodiments, before radiation therapy, the first detection assembly may be controlled to receive the photon signal generated by the radioactive isotope within the object by controlling the independent rotation of the first rotatable part relative to the second rotatable part (e.g., rotation of the first rotatable part, stationary second rotatable part, or rotation of the first rotatable part and the second rotatable part at different speeds), to determine the first image of the target region of the object. In some embodiments, before radiation therapy, the first detection assembly may be controlled to receive the photon signal generated by the radioactive isotope within the object by controlling the first rotatable part to be stationary, to determine the first image of the target region of the object.

In some embodiments, before radiation therapy, the first rotatable part may be controlled to rotate independently of the second rotatable part, allowing one of the at least one imaging source to emit an imaging beam toward the object to determine the second image of the target region of the object. In some other embodiments, before radiation therapy, the first rotatable part may be controlled to remain stationary, allowing one of the at least one imaging source to emit an imaging beam toward the object to determine the second image of the target region of the object. In some other embodiments, before radiation therapy, the first rotatable part may be controlled to rotate synchronously with the second rotatable part, allowing one of the at least one imaging source to emit an imaging beam toward the object to determine the second image of the target region of the object. At this time, if the imaging source (e.g., a CT imaging source or a DR imaging source) and/or the corresponding detector (e.g., a CT detector or a DR detector) are obscured by the first detection assembly, the first detection assembly may be moved (e.g., along the axis of the frame 111) to ensure that at least a portion of the imaging beam emitted by one of the at least one imaging source is not obscured by the first detection assembly, for example, by switching the first detection assembly from the first position to the second position.

In some embodiments, precise positioning of the target region to be treated or determining/adjusting a treatment plan for the object may be achieved based on the first image and/or the second image. For example, positioning/location of the object and the treatment plan for the target region of the object may be determined or adjusted using a fused image of the object's PET image/SPECT image and CT image/MR image. Furthermore, real-time guidance of radiation therapy may be performed based on the fused image. In some embodiments, after the precise positioning of the target region of the object is completed, the first rotatable part and the second rotatable part may be locked to achieve synchronous rotation, by which the radiation source can emit the therapeutic beam to treat the target region of the object.

In some embodiments, during radiation therapy, the first detection assembly may be located at the first position and may receive the photon signal generated by the radioactive isotope within the object to generate the first image of the target region of the object. In some embodiments, during radiation therapy, the first detection assembly may refrain from being obscured by the imaging source and the corresponding imaging assembly (e.g., by moving the CT imaging source and the CT detector relative to the first rotatable part to move away from the position of the first detection assembly, avoiding obstruction of the first detection assembly), to receive the photon signal generated by the radioactive isotope within the object, generating the first image of the target region of the object. In some embodiments, during radiation therapy, the first detection assembly is located at the second position, allowing one of the at least one imaging source to emit an imaging beam toward the object to obtain the second image of the target region of the object. In some embodiments, real-time guidance of the radiation source toward the target region of the object may be achieved based on the first image and/or the second image during radiation therapy. In some embodiments, the first image or the second image may be shown to a user for observation of the positioning of the target region of the object. In some embodiments, during radiation therapy, the first detection assembly may be switched from the first position to the second position, enabling conventional radiation therapy by controlling the treatment head to emit a therapeutic beam toward the target region of the object. By placing the first detection assembly at the second position, radiation from the therapeutic beam emitted by the radiation source to the first detection assembly may be avoided, thereby increasing the lifespan of the first detection assembly and consequently enhancing the lifespan of the image-guided radiation therapy system.

Further details regarding a radiation therapy method may be found elsewhere (e.g., in FIG.s 11-12 and related descriptions thereof) in the present disclosure, and are not reiterated here.

In some embodiments, other treatment assemblies (e.g., the collimation part and the therapeutic beam detector) may be mounted on the first rotatable part. The therapeutic beam detector, for example, an electronic portal imaging device (EPID), may be used to receive at least a portion of the therapeutic beam emitted by the radiation source.

In some embodiments, other treatment assemblies (e.g., the collimation part and the therapeutic beam detector) may also be mounted on the second rotatable part. In this case, the first rotatable part may include an opening. After the precise positioning of the target region of the object is completed, the first rotatable part may be rotated to align the treatment head with the opening. Subsequently, at least a portion (referred to as a second rotating part) of the second rotatable part is lowered (such as radially along the second rotatable part), and then the first rotatable part and the second rotatable part are locked. After that, the radiation source emits the therapeutic beam to treat the target region of the object. The second rotating part (e.g., the upper treatment head) may move radially (along the z-axis direction in FIG. 1), and the second rotating part (e.g., the upper treatment head) may be lowered as a whole. As used herein, "lowered" refers to being moved radially toward the center of rotation. For example, by placing the upper treatment head of the second rotatable part on a slider, the upper treatment head of the second rotatable part may be lowered by moving the slider radially (along the negative z-axis direction in FIG. 1) toward the center of rotation (the origin in FIG.1). In some embodiments, a movable part in the second rotatable part may be lowered. The movable part in the second rotatable part may include one or more of the collimation parts, such as the multi-leaf collimator, the gantry, the ionization chamber, a primary collimator, and a radiation source emitting the therapeutic beam, a target, the microwave part or the acceleration part, or a combination thereof. In some embodiments, at least one part in the movable part of the second rotatable part is moved into the opening. For example, a part to be moved may be mounted on a support and provided with a slider and a corresponding drive mechanism for moving the support along the slider to radially move the at least one part in the movable part. For example, the multi-leaf collimator and the gantry may be moved into the opening. As another example, the multi-leaf collimator, the gantry, the primary collimator, and the ionization chamber may be moved into the opening. As yet another example, the multi-leaf collimator, the gantry, the primary collimator, the ionization chamber, and the radiation source emitting the therapeutic beam may be moved into the opening. In some embodiments, a distance between the radiation source emitting the therapeutic beam and the center of the treatment head may be in the range of 90-110 centimeters (e.g., 100 centimeters).

FIG.s 2(a) to 6(b) are schematic diagrams illustrating schematic diagrams illustrating structures of exemplary image-guided radiation therapy systems according to some embodiments of the present disclosure. The image-guided radiation therapy systems 200 to 600 correspond to the medical device 110 in FIG. 1.

As shown in FIG.s 2(a) or 2(b), the image-guided radiation therapy system 200 (or the medical device 110) may include a first detection assembly 210, a radiation source 220, a first rotatable part 230, and a second rotatable part 240.

In some embodiments, the first detection assembly 210 may be mounted on the first rotatable part 230 to receive a photon signal generated by a radioactive isotope within an object, generating a first image of a target region of the object. In some embodiments, the radiation source 220 may be mounted on the second rotatable part 240 to emit a therapeutic beam to the target region of the object.

As shown in FIG.s 2(a) or 2(b), in some embodiments, a treatment assembly may also include a collimation part 223 and a therapeutic beam detector 225. The collimation part 223 is used to limit the radiation range (e.g., angle, shape) of a therapeutic beam emitted by the radiation source 220, and the therapeutic beam detector 225 is used to receive at least a portion of the therapeutic beam.

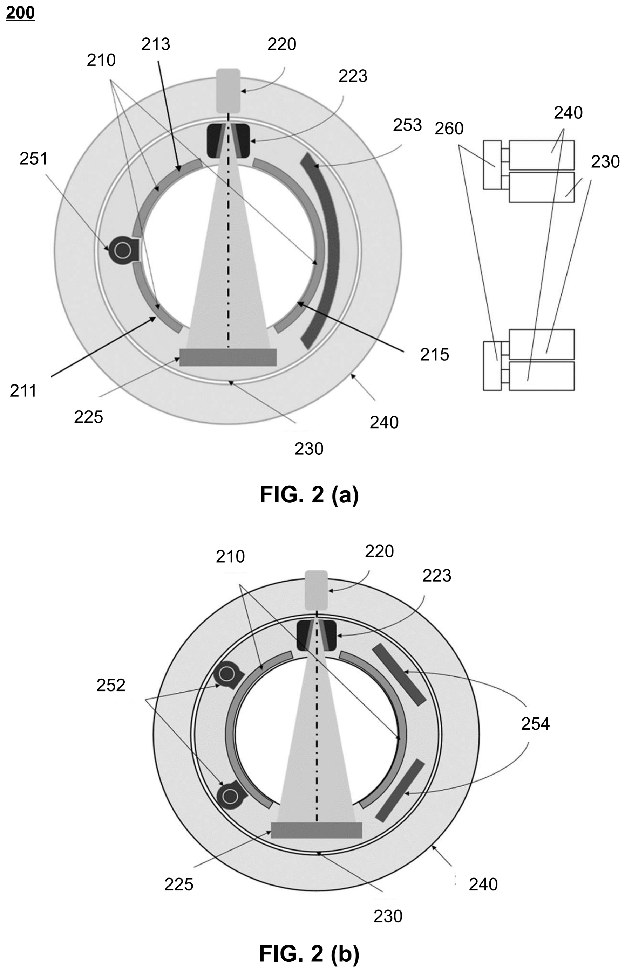

In some embodiments, the image-guided radiation therapy system 200 may also include a fixed portion 260. The fixed portion 260 may be connected to the first rotatable part 230 and the second rotatable part 240. For example, the first rotatable part and the second rotatable part may be connected to the fixed portion 260 via a first bearing and a second bearing, respectively. As another example, the first rotatable part 230 may be connected to the fixed portion 260 via the first bearing, and the second rotatable part 240 may be connected to the first rotatable part 230 via the second bearing. The first rotatable part 230 and the second rotatable part 240 are configured to move independently. For example, the first rotatable part 230 may rotate around a first ring, and the second rotatable part 240 may rotate around a second ring. Wherein, the first ring is different from the second ring, and the first ring and the second ring are coplanar. For example, the first ring and the second ring are two concentric rings along a circumference shown in FIG. 2(a), according to some embodiments of the present disclosure.

In some embodiments, the image-guided radiation therapy system 200 may also include at least one imaging source. In some embodiments, the at least one imaging source may include one or more CT imaging sources and/or one or more DR imaging sources. For example, as shown in FIG. 2(a), the at least one imaging source may include a CT tube 251. As another example, as shown in FIG. 2(b), the CT imaging source may include two DR tubes 252.

In some embodiments, the image-guided radiation therapy system 200 may also include other imaging assemblies, such as one or more detectors corresponding to the at least one imaging source. For example, as shown in FIG. 2(a), the detector corresponding to the imaging source may be a CT detector 253, and the CT detector 253 is arranged to face the CT tube 251. As another example, as shown in FIG. 2(b), the detectors corresponding to the imaging source may be two DR detectors 254, and the two DR detectors 254 are respectively arranged to face the two DR tubes 252.

The tubes (e.g., the CT tube 251, the DR tubes 252) may be used to emit an imaging beam to the target region of the object, and the one or more detectors corresponding to the tubes (e.g., the CT detector 253, the DR detectors 254) are used to detect at least a portion of the imaging beam to generate a second image of the object. For example, the CT detector 253 may be used to detect the imaging beam emitted by the CT tube 251 towards the target region of the object, generating the second image of the object. As another example, the DR detectors 254 may be used to receive the imaging beam passing through the target region of the object emitted by the DR tubes 252, generating the second image of the object.

In some embodiments, the first detection assembly 210, the radiation source 220, the collimation part 223, the therapeutic beam detector 225, the first rotatable part 230, the second rotatable part 240, the at least one imaging source (e.g., the CT tube 251, the DR tube 252s) and corresponding detectors (e.g., the CT detector 253, the DR detector 254s) may be located on the same plane (e.g., a vertical plane parallel to the paper in the FIGs). At this time, the first detection assembly 210 is positioned at a first position (e.g., the arc position corresponding to 210 in FIG. 2(a)).

] In some embodiments, the first detection assembly 210 and the at least one imaging source (e.g., the CT tube 251 or the DR tubes 252) may be located on the same circumference of the first rotatable part 230. In some embodiments, the first detection assembly 210 may include three sets of detectors. Two sets of detectors among the at least three sets of detectors are arranged to face other detectors of the at least three sets of detectors, and the at least one imaging source is mounted between the two sets of detectors. For example, as shown in FIG. 2(a), the first detection assembly 210 may include three sets of detectors, where a first set of detectors 215 is located to the right (as indicated in FIG. 2(a)) of a line (e.g., an imaginary straight line in FIG. 2(a)) connecting the radiation source 220 and the therapeutic beam detector 225, and a second set of detectors 211 and a third set of detectors 213 are located to the left of the line (as indicated in FIG. 2(a)). The first set of detectors 215 may be arranged to face the second set of detectors 211 and the third set of detectors 213, with the CT tube 251 located between the second set of detectors 211 and the third set of detectors 213.

In some embodiments, the first detection assembly 210 may include two sets of detectors arranged to face each other, and the at least one imaging source and corresponding detectors may be mounted inside or outside a region formed by the two sets of detectors. For example, as shown in FIG. 2(b), the first detection assembly 210 may include two sets of detectors: a first set of detectors and a second set of detectors. The first set of detectors may be located to the right (as indicated in FIG. 2(b)) of a line (e.g., an imaginary straight line in FIG. 2(b)) connecting the radiation source 220 and the therapeutic beam detector 225, and the second set of detectors may be located to the left of the line (as indicated in FIG. 2(b)). The first set of detectors may be arranged to face the second set of detectors, with the DR tubes 252 and the DR detectors 254 located outside a circular region formed by the first set of detectors and the second set of detectors.

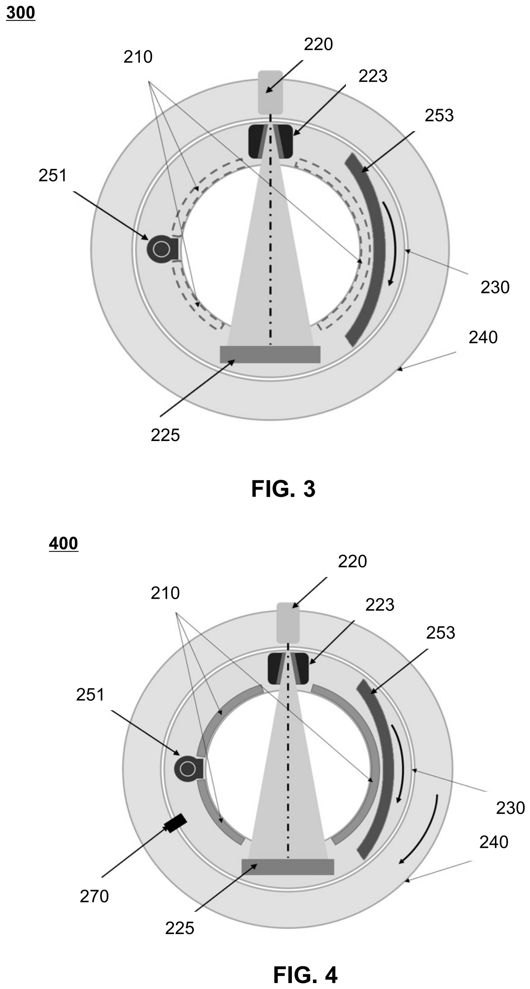

FIG. 3 is a schematic diagram illustrating a structure of an exemplary image-guided radiation therapy system according to some embodiments of the present disclosure. Similar to the image-guided radiation therapy system 200 shown in FIG.s 2(a) or 2(b), the image-guided radiation therapy system 300 (or the medical device 110) may include a first detection assembly 210, a radiation source 220, a collimation part 223, a therapeutic beam detector 225, a first rotatable part 230, a second rotatable part 240, at least one imaging source (e.g., a CT tube 251), and other imaging assemblies (e.g., a CT detector 253).

In FIG. 3, the first rotatable part 230 and the second rotatable part 240 may rotate independently of each other. For example, the first rotatable part 230 may rotate while the second rotatable part 240 remains stationary, or the first rotatable part 230 may remain stationary while the second rotatable part 240 rotates. In some embodiments, the first rotatable part 230 and the second rotatable part 240 may rotate in different directions. For example, the first rotatable part 230 may rotate clockwise along a direction indicated by the arrow in FIG. 3, while the second rotatable part 240 may rotate counterclockwise along an opposite direction indicated by the arrow in FIG. 3.

In some embodiments, before administering radiation therapy to an object, the imaging source (e.g., the CT tube 251) may emit an imaging beam toward the object. Based on at least a portion of the imaging beam detected by a second detection assembly (e.g., a CT detector 253), a second image of a target region of the object is generated. At this point, the first detection assembly 210 may be moved out of an isocentric plane (e.g., a plane defined by the dashed arc in FIG. 3) to a second position to avoid obscuring the imaging beam. In some embodiments, the first detection assembly 210 may move a preset distance along a rotational axis direction of the image-guided radiation therapy system 300 (e.g., the y-axis direction in FIG. 1) to reach the second position. In some embodiments, the first detection assembly 210 may be partially or entirely moved out of the isocentric plane.

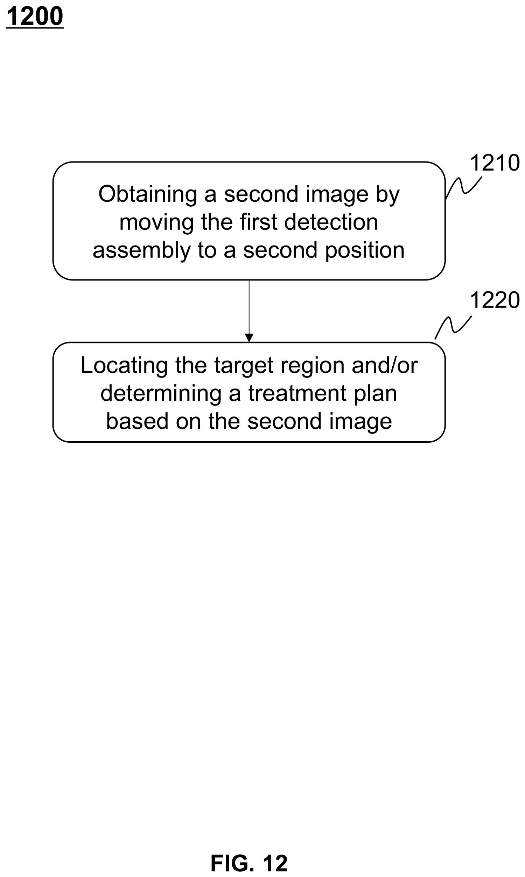

In some embodiments, the target region of the object may be positioned and/or a treatment plan adjusted based on a first image and/or the second image. For example, beam parameters of a treatment plan, such as a beam angle, a collimator angle, a field size, a gantry lock position, etc., for the current object may be determined based on the target region in the first image, the second image, or a third image determined (e.g., through fusion or registration) based on the first and second images. This ensures that a radiation beam of a therapeutic beam covers the target region while avoiding harm to other tissues in a human body. Further details on image-guided radiation therapy based on the first image or the second image may be found in FIG. 12 and related descriptions thereof, which are not repeated here.

FIG. 4 is a schematic diagram illustrating a structure of an exemplary image-guided radiation therapy system according to some embodiments of the present disclosure. Similar to the image-guided radiation therapy system 200 shown in FIG.s 2(a) or 2(b) and the image-guided radiation therapy system 300 shown in FIG. 3, the image-guided radiation therapy system 400 (or medical device 110) may include a first detection assembly 210, a radiation source 220, a collimation part 223, a therapeutic beam detector 225, a first rotatable part 230, a second rotatable part 240, at least one imaging source (e.g., a CT tube 251), and other imaging assembly (e.g., a CT detector 253).

Furthermore, the image-guided radiation therapy system 400 may also include a locking part 270. The locking part 270 may be used to lock the first rotatable part 230 and the second rotatable part 240, enabling synchronous rotation of the first rotatable part 230 and the second rotatable part 240.

In some embodiments, after positioning a target region of an object (e.g., based on position information of the target region determined based on a first image and/or a second image, and controlling the bed 115 to position the target region of the object in the hole 116), during a radiation therapy process, the first detection assembly 210 may be positioned at a first position by locking the first rotatable part 230 and the second rotatable part 240 using the locking part 270. Subsequently, the first rotatable part 230 and/or the second rotatable part 240 may be rotated (e.g., clockwise along the direction indicated by the arrow in FIG. 4) based on a radiation treatment plan. This allows the first detection assembly to receive a photon signal generated by a radioactive isotope within the object, generating the first image of the target region. Simultaneously, based on the first image, the radiation source 220 may emit a therapeutic beam to the target region of the object, facilitating real-time image-guided radiation therapy.

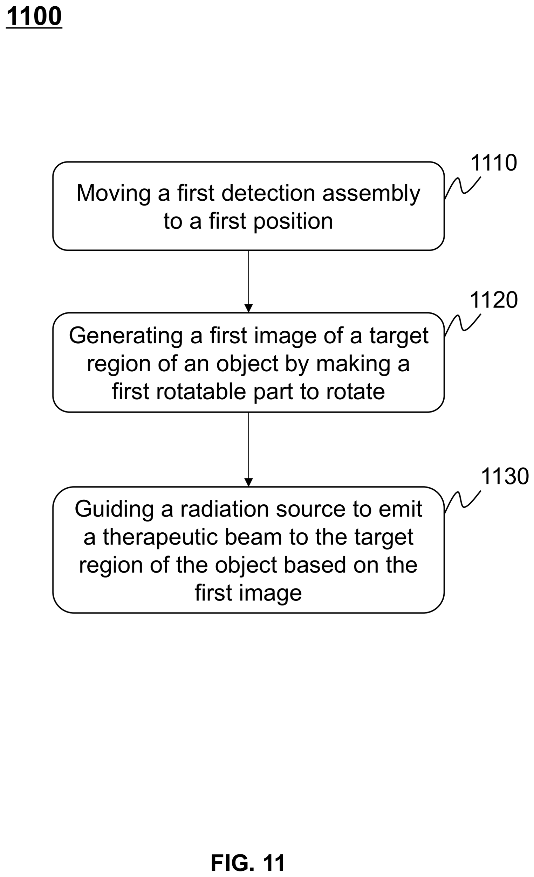

In some embodiments, real-time adjustments to the therapeutic beam emitted by the radiation source 220 may be made based on the first image and/or second image, enabling tracking and positioning of the radiation therapy beam on the target region of the object. For example, during the radiation therapy process, the first image and/or the second image may be generated at preset time intervals or in real time. Based on a third image determined based on the first image, or the second image, or the first and the second images, adjustments to the angle of the therapeutic beam emitted by a treatment head may be made in real time. This ensures that at least a portion of the therapeutic beam aligns with the target region of the object. Further details on real-time image-guided radiation therapy may be found in FIG. 11 and related descriptions thereof, which are not repeated here.

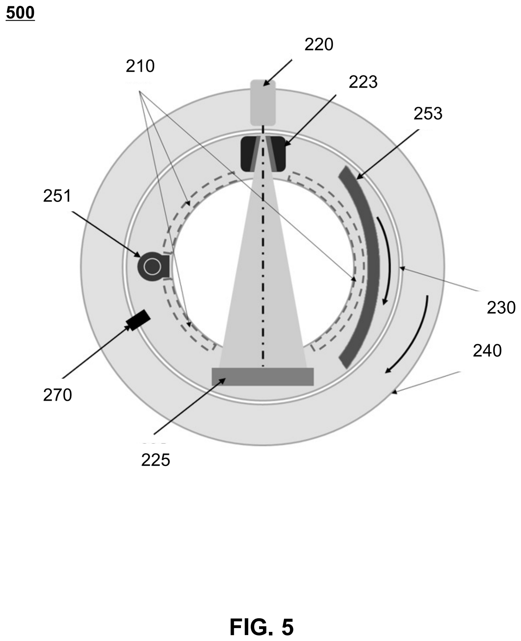

FIG. 5 is a schematic diagram illustrating a structure of an exemplary image-guided radiation therapy system according to some embodiments of the present disclosure. Similar to the image-guided radiation therapy system 400 shown in FIG. 4, the image-guided radiation therapy system 500 (or the medical device 110) may include a first detection assembly 210, a radiation source 220, a collimation part 223, a therapeutic beam detector 225, a first rotatable part 230, a second rotatable part 240, at least one imaging source (e.g., a CT tube 251), other imaging assemblies (e.g., a CT detector 253), and a locking part 270.

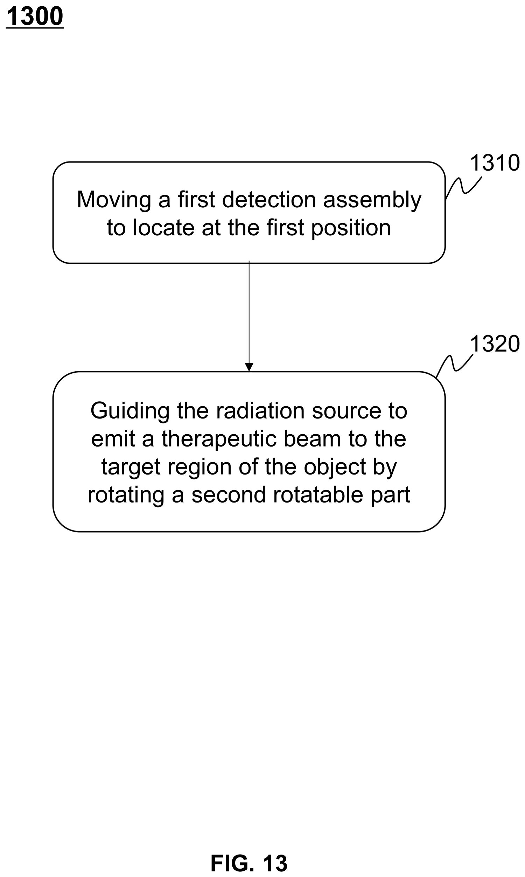

In some embodiments, after positioning a target region of an object, during the radiation therapy process, the locking part 270 may be used to lock the first rotatable part 230 and the second rotatable part 240, such that the first detection assembly 210 moves out of an isocentric plane to a second position. Subsequently, based on a radiation treatment plan, the first rotatable part 230 and the second rotatable part 240 may be rotated (e.g., clockwise along a direction indicated by the arrow in FIG. 5), enabling the radiation source 220 to emit a therapeutic beam for the radiation therapy of the target region of the object. For further details on conventional radiation therapy, reference may be made to FIG. 13 and related descriptions thereof, which is not reiterated here.

In some embodiments, after positioning the target region of the object (e.g., based on position information of the target region determined based on a first image and/or a second image, and controlling the bed 115 to position the target region of the object in the hole 116), during the radiation therapy process, the first detection assembly 210 may be positioned at a first position by locking the first rotatable part 230 and the second rotatable part 240 using the locking part 270. Subsequently, based on the radiation treatment plan, the first rotatable part 230 and/or the second rotatable part 240 may be rotated (e.g., clockwise along the direction indicated by the arrow in FIG. 5), enabling the at least one imaging source (e.g., the CT tube 251 or DR tube 252) to emit an imaging beam towards the target region of the object. This generates the second image of the target region of the object. Simultaneously, based on the second image, the radiation source 220 may emit the therapeutic beam to the target region of the object, facilitating real-time image-guided radiation therapy.

FIG.s 6(a)-6(b) are schematic diagrams illustrating structures of an exemplary image-guided radiation therapy system according to some embodiments of the present disclosure. The image-guided radiation therapy system 600 (or the medical device 110) may include a first detection assembly 210, a radiation source 220, a collimation part 223, a therapeutic beam detector 225, a first rotatable part 230, a second rotatable part 240, at least one imaging source (e.g., a CT tube 251), other imaging assemblies (e.g., a CT detector 253), and a locking part 270.

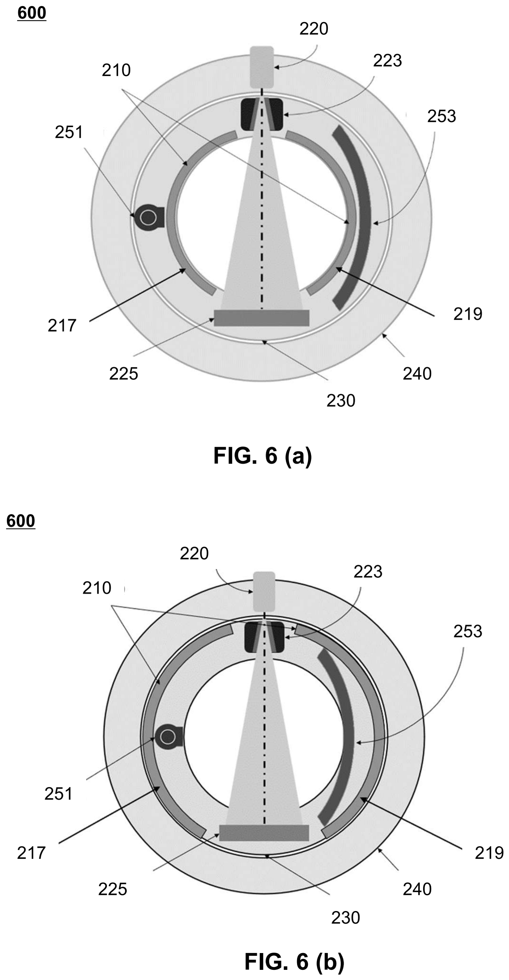

As shown in FIG. 6(a), in some embodiments, the first detection assembly 210 may include two sets of detectors arranged to face each other. In some embodiments, when the first detection assembly 210 is in a first position, the imaging source (e.g., CT tube 251) may be obscured by one detector (e.g., a left-side detector 217 in FIG. 6(a)) of the two sets of detectors. In some embodiments, when the first detection assembly 210 is in the first position, a second detection assembly (e.g., the CT detector 253) may be obscured by one (e.g., a right-side detector 219 in FIG. 6(a)) of the two sets of detectors.

As shown in FIG. 6(b), in some embodiments, the first detection assembly 210 may include two sets of detectors to face each other. In some embodiments, when the first detection assembly 210 is in the first position, one detector (e.g., a left-side detector 217 in FIG. 6(b)) of the two sets of detectors may be obscured by the imaging source (e.g., the CT tube 251). In some embodiments, when the first detection assembly 210 is in the first position, one detector (e.g., ta left-side detector 217 in FIG. 6(b)) of the two sets of detectors may be obscured by a detector (e.g., the CT detector 253) corresponding to the imaging source.

In some embodiments, the imaging source and the detector corresponding to the imaging source may be arranged to face each other. For example, as shown in FIG.s 2(a) and 3-6(b), the CT tube 251 may be arranged to face the CT detector 253, and in FIG. 2(b), the DR tubes 252 may be arranged to face the DR detectors 254. "Arranged to face" here refers to a functioning side of a component being arranged to face a functioning side of another component. For example, a side of the CT tube 251 emitting an imaging beam is positioned to face a side of the CT detector 253 receiving the imaging beam. As another example, a side of the first set of detectors 215 receiving a photon signal is positioned to face the sides of the second set of detectors 211 and the third set of detectors 213 that receive the photon signal.

It is understood that the relative positions of the components shown in FIG.s 2(a)-6(b) are for illustration purposes only, and do not intend to limit the scope of the present disclosure. In some embodiments, these components may have other configurations deemed reasonable by those skilled in the art. For example, the CT tube 251 may be located above or below the first rotatable part 230. In some embodiments, the image-guided radiation therapy systems 200-600 (or the medical device 110) may also include other therapy or imaging assembly, such as an X-ray source, specifics of which are not restricted by the present disclosure.

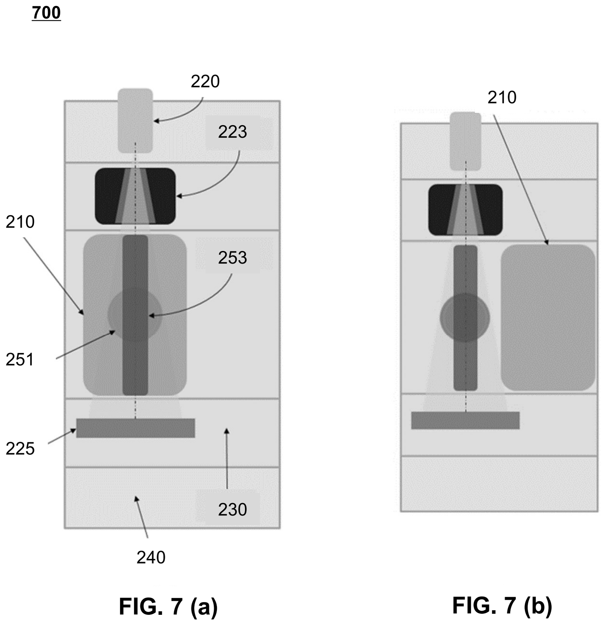

FIGs. 7 (a) and (b) are schematic diagrams illustrating side views of an exemplary image-guided radiation therapy system according to some embodiments of the present disclosure. Similar to the image-guided radiation therapy systems 200-600 shown in FIG.s 2(a)-6(b), the image-guided radiation therapy system 700 (or the medical device 110) may include a first detection assembly 210, a radiation source 220, a collimation part 223, a therapeutic beam detector 225, a first rotatable part 230, a second rotatable part 240, at least one imaging source (e.g., a CT tube 251 or a DR tube 252), and other imaging assembly (e.g., a CT detector 253 or a DR detector 254).

For illustrative purposes only, when viewed from a side of the image-guided radiation therapy system (e.g., image-guided radiation therapy systems 200-600) or the medical device 110, when the first detection assembly 210 is in a first position, as shown in FIG. 7(a), the first detection assembly 210, the radiation source 220, the collimation part 223, the therapeutic beam detector 225, the CT tube 251 (or the DR tube 252), and the CT detector 253 (or the DR detector 254) may be located on a same line (e.g., a first line). When the first detection assembly 210 is in a second position, as shown in FIG. 7(b), the radiation source 220, the collimation part 223, the therapeutic beam detector 225, the CT tube 251 (or the DR tube 252), and the CT detector 253 (or the DR detector 254) are located on the first straight line, while the first detection assembly 210 is on a second straight line. At this point, during CT/DR imaging or conventional radiation therapy of an object, since the first detection assembly 210, the radiation source 220, the CT tube 251 (or the DR tube 252), and the CT detector 253 (or the DR detector 254) are located on different straight lines, the first detection assembly 210 cannot obscure an imaging beam and does not interfere with a therapeutic beam, thus having no impact on CT/DR imaging or radiation therapy.

It is understood that the second position of the first detection assembly 210 in FIG. 7 is for illustrative purposes only, and does not intend to limit the scope of the present disclosure. In some embodiments, the second position may be any other position that does not affect CT/DR imaging and radiation therapy, on which the present disclosure does not impose restrictions.

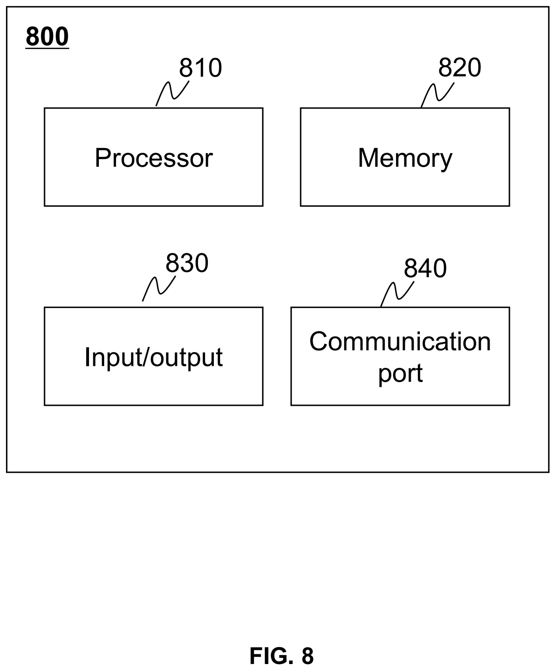

FIG. 8 is a schematic diagram illustrating hardware and/or software components of an exemplary computing device according to some embodiments of the present disclosure. In some embodiments, the processing device 140 may be implemented on a computing device 800. As shown in FIG. 8, the computing device 800 may include a processor 810, a memory 820, an input/output 830, and a communication port 840.

The processor 810 may execute a computer instruction (program code) and perform the functions of the processing device 140 as described in the present disclosure. The computer instruction may include a routine, a program, an object, a component, a data structure, a process, a module, and a function that performs a specific function described in the present disclosure. For example, the processor 810 may process data obtained from the medical device 110, the at least one terminal device 130, the storage device 150, or any other component of the image-guided radiation therapy system 100. In some embodiments, the processor 810 may include a microcontroller, a microprocessor, a reduced instruction set computer, an application-specific integrated circuit, a special instruction set processor, a central processing unit, a graphics processing unit, a physical processing unit, a microcontroller unit, a digital signal processor, a field-programmable gate array, a high-level reduced instruction set computer system, a programmable logic device, or any combination thereof, capable of executing one or more of the above functions.

For ease of illustration, only one processor is described in FIG. 8, which may also include a plurality of processors. Therefore, the operations and/or method steps described in the present disclosure as being performed by one processor may also be collectively or individually performed by a plurality of processors. For example, if in the present disclosure, the processor of the computing device 800 performs steps A and B, it should be understood that steps A and B may also be collectively or independently executed by two different central processing units and/or processors of the computing device 800 (e.g., a first processor performs step A, and a second processor performs step B, or the first and second processors collectively perform steps A and B).

The memory 820 may store data/information obtained from the medical device 110, the at least one terminal device 130, the storage device 150, or any other component of the image-guided radiation therapy system 100. In some embodiments, the memory 820 may include a high-capacity memory, a removable memory, a volatile read-write memory, a read-only memory, or any combination thereof. Exemplary high-capacity memory may include a disk, an optical disk, a solid-state disk, or the like. In some embodiments, the memory 820 may store one or more programs and/or instructions to execute exemplary methods described in the present disclosure.

The input/output 830 may input or output a signal, data, or information. In some embodiments, the input/output 830 may enable user interaction with the processing device 140. In some embodiments, the input/output 830 may include an input device and an output device. Exemplary input devices may include a keyboard, a mouse, a touchscreen, a microphone, or combinations thereof. Exemplary output devices may include a display device, a speaker, a printer, a projector, or combinations thereof. Exemplary display devices may include a liquid crystal display (LCD) display, a light emitting diode (LED) display, a tablet display, a curved screen, a television device, a cathode ray tube, or combinations thereof.

The communication port 840 may connect to a network (e.g., the network 120) to facilitate data communication. The communication port 840 may establish a connection between the processing device 140 and the medical device 110, the at least one terminal device 130, or the storage device 150. The connection may be a wired connection, a wireless connection, or a combination thereof, enabling data transmission and reception. The wired connection may include a cable, an optical fiber, a telephone line, or any combination thereof. The wireless connection may include Bluetooth, Wi-Fi, WiMax, WLAN, ZigBee, a mobile network (e.g., 3G, 4G, 5G), or combinations thereof. In some embodiments, the communication port 840 may be a standardized communication port, such as RS232, RS485, or the like. In some embodiments, the communication port 840 may be a specially designed communication port, for example, designed according to a medical digital imaging and communication protocol.

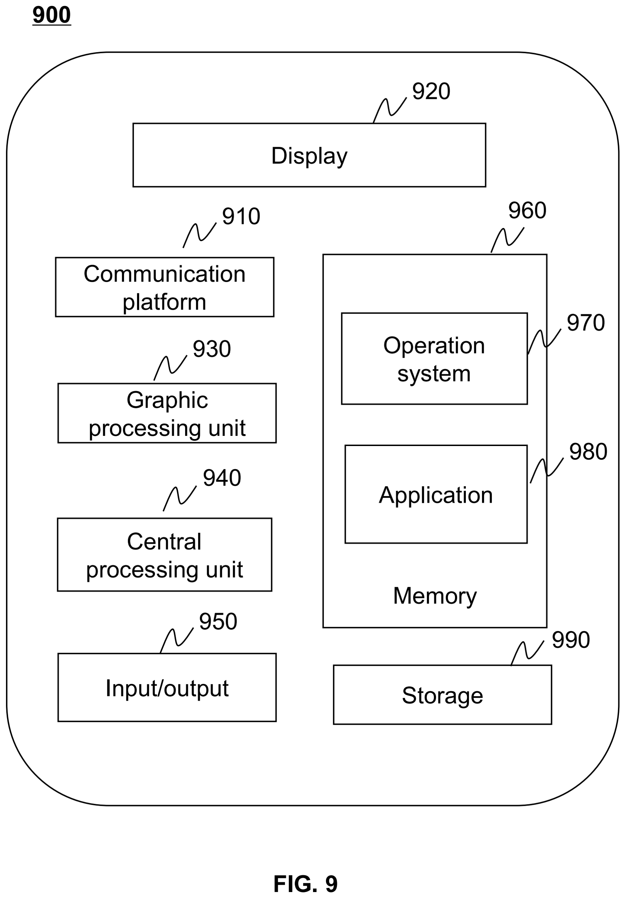

FIG. 9 is a schematic diagram illustrating hardware and/or software components of an exemplary terminal device according to some embodiments of the present disclosure. In some embodiments, the at least one terminal device 130 may be implemented on a mobile device 900. As shown in FIG. 9, the mobile device 900 may include a communication platform 910, a display 920, a graphics processing unit 930, a central processing unit 940, an input/output 950, a memory 960, and a storage 990. In some embodiments, any other suitable components, including but not limited to a system bus or a controller (not shown in the figure), may also be included in the mobile device 900. In some embodiments, an operating system 970 (e.g., iOS, Android, Windows Phone, etc.) and one or more applications 980 may be downloaded from the storage 990 to the memory 960 for execution by the central processing unit 940. The applications 980 may include a browser or any other suitable mobile application for receiving and presenting information related to radiation therapy or other information from the processing device 140. User interaction with information flow may be achieved through the input/output 950 and provided to the processing device 140 and/or other components of the image-guided radiation therapy system 100 via the network 120.

To implement the functions of two or more modules and units as described in the present disclosure, a computer hardware platform may be used as a hardware platform for one or more components described in the present disclosure. The hardware components, operating system, and programming language of such computers are essentially conventional and may be understood by those skilled in the art to adapt to the radiation therapy described in the present disclosure. A computer with a user interface element may be used to implement a personal computer or another type of workstation or terminal device and, if properly programmed, may also serve as a server. Those skilled in the art are believed to be familiar with the structure, programming, and general operation of such computer devices, so the drawing is self-explanatory.

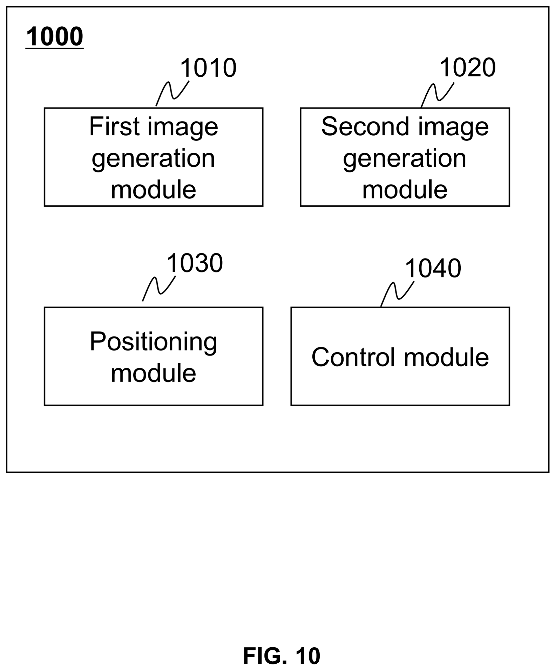

FIG. 10 is a modular schematic diagram illustrating an exemplary image-guided radiation therapy system according to some embodiments of the present disclosure. As shown in FIG. 10, in some embodiments, an image-guided radiation therapy system 1000 may include a first image generation module 1010, a second image generation module 1020, a positioning module 1030, and a control module 1040. In some embodiments, the image-guided radiation therapy system 1000 may be implemented on the processing device 140 or the medical device 110.

The first image generation module 1010 may be configured to determine a first image of a target region of an object by causing a first detection assembly in the medical device 110 to receive a photon signal generated by a radioactive isotope within the object.

The second image generation module 1020 may be configured to determine a second image of the target region of the object by making at least one imaging source in the medical device 110 emit an imaging beam toward the object.

The positioning module 1030 may be configured to position the target region of the object based on the first image and/or the second image of the object in real time.

In some embodiments, the positioning module 1030 may register the first image and/or the second image with a plan image used to develop a treatment plan to position the target region of the object and/or adjust the treatment plan. For example, the positioning module 1030 may determine position information of the target region of the object based on a registration result. Then, the target region may be placed at an isocenter of a radiotherapy device in the radiation therapy system, or a therapeutic beam may be adjusted to align with the target region of the object.

The control module 1040 may be configured to emit a therapeutic beam from a radiation source to the target region of the object. The therapeutic beam may deliver radiation therapy to the target region. In some embodiments, a movement of an organ (e.g., heart motion, respiratory motion, blood flow, muscle movement, pancreatic secretion activity, bladder movement, and movement of the rectum and digestive system) within or near the target region of the object may cause a change in a position of the target region.

In some embodiments, the control module 1040 may monitor, in real time, the change in the position of the target region based on the first image or the second image of the object, enabling timely adjustments of the therapeutic beam to align with the target region and ensure treatment accuracy.

In some embodiments, when a movement or a change in the target region is detected, the control module 1040 may adjust the radiation direction of the therapeutic beam. For example, the control module 1040 may adjust an upper treatment head and/or a collimator to align the object with the position of the moving or changing target region. In some embodiments, when a movement or a change in the target region is detected, the control module 1040 may modify the position of the object. For example, the control module 1040 may pause radiation of the therapeutic beam, then adjust a position of the target region relative to the therapeutic beam to align the therapeutic beam with the target region. After adjusting the radiation of the therapeutic beam or the position of the object, the radiation of the therapeutic beam may be resumed. In some embodiments, when a movement or a change in the target region is detected, the radiation source may terminate the radiation of the therapeutic beam. In some embodiments, control module 1040 may generate a notification based on the detected movement or change in the target region. In some embodiments, the notification may include information about the movement or change in the target region and may be in a form of text, video, audio, etc.

According to the systems and methods described in the present disclosure, the control module 1040 may automatically generate and/or analyze an image to record the process of radiation therapy, monitor the position of the target region, assess changes in the position of the target region, and/or determine how to proceed with the radiation therapy (e.g., continue the radiation therapy as planned, continue the radiation therapy with an adjusted plan, terminate the radiation therapy, etc.). In some embodiments, a user input (e.g., from a doctor) may be used to semi-automatically perform the monitoring, assessment, and/or adjustment. For example, the control module 1040 may send the first image to be presented on the at least one terminal device 130 (e.g., a display), allowing the user to analyze the first image and provide an instruction on how to proceed with the treatment plan (e.g., continue the radiation therapy as planned, continue the radiation therapy with an adjusted plan, terminate the radiation therapy, etc.). As another example, the control module 1040 may first analyze the image, determine if there are any changes in the target region, and how much is changed. The control module 1040 may then determine whether any adjustments to the treatment plan are needed. If a change in the target region or an adjustment required in the treatment plan is within a threshold, the control module 1040 may automatically adjust. Meanwhile, the control module 1040 may generate a notification to inform the user. If the change in the target region or the adjustment required in the treatment plan exceeds the threshold, the control module 1040 may generate, for example, a notification, seeking an instruction from the user on how to proceed with radiation therapy.

It should be noted that the descriptions of system 1000 above are for convenience of illustration and do not limit the present disclosure to the scope of the disclosed embodiments. It is understood that, for those skilled in the art, various combinations of devices/modules may be made without departing from the principles of the system. After understanding the principles of the system, those skilled in the art may arbitrarily combine various devices/modules or constitute subsystems connected to other devices/modules. For example, the first image generation module 1010, the second image generation module 1020, the positioning module 1030, and the control module 1040 disclosed in FIG. 10 may be different modules within a device (e.g., the processing device 140), or a single module implementing the functions of two or more of the above modules. For example, the positioning module 1030 and the control module 1040 may be two separate modules or a single module that simultaneously positions the target region of the object and emits the therapeutic beam toward the target region. As another example, each device may have a corresponding storage module or share a common storage module. Such variations are within the scope of the present disclosure.

FIG. 11 is a flowchart illustrating an exemplary process of an image-guided radiation therapy method according to some embodiments of the present disclosure.

In some embodiments, an image-guided radiation therapy method 1100 for an image-guided radiation therapy system (e.g., image-guided radiation therapy systems 200 to 600) may be executed by a processing device 140 (e.g., the processor 810 in FIG. 8, the processing device 140 in FIG. 1) or an image-guided radiation therapy system 1000. For example, the image-guided radiation therapy method 1100 may be stored in a storage device (e.g., the storage device 150, the memory 820, the storage 990) in the form of a program or an instruction, and when the processing device 140 executes the program or the instruction, the method 1100 may be implemented. As shown in FIG. 11, in some embodiments, during radiation therapy, the processing device 140 may cause a first detection assembly to obtain real-time a first image of an object based on the first image, providing real-time guidance for the radiation therapy.

In operation 1110, the first detection assembly is positioned at a first position.

In some embodiments, the processing device 140 may position the first detection assembly at the first position by sending a control instruction to the medical device 110. For example, after the locking part 270 locks the first rotatable part 230 and the second rotatable part 240, a control part of the medical device 110 may control a drive mechanism to position the first detection assembly 210 at the first position based on the control instruction. In some embodiments, the first rotatable part and the second rotatable part may be manually locked and/or the first detection assembly may be manually positioned. For example, an operator or medical personnel of the medical device 110 may manually turn the locking part 270 on the medical device 110 to lock the first rotatable part and the second rotatable part. As another example, a user may manually position the first detection assembly 210 by operating a control button of the medical device 110. In some embodiments, the first detection assembly of the radiation therapy system (e.g., radiation therapy systems 200 to 600) may be positioned at the first position in an initial state (e.g., after system startup) or in a standby state of the system.

In operation 1120, the first image of a target region of an object is generated by making the first rotatable part rotate.

In some embodiments, after placing the object within a bore (e.g., the bore 116) of the image-guided radiation therapy system, the processing device 140 may, based on the treatment plan, control the first rotatable part and the second rotatable part of the image-guided radiation therapy system (e.g., the image-guided radiation therapy systems 200 to 600 or the medical device 110) to rotate synchronously (i.e., keeping the first rotatable part and the second rotatable part relatively stationary). This allows the first detection assembly to receive a photon signal generated by a radioactive isotope within the object, thereby generating (determining) the first image (e.g., a three-dimensional image) of the target region of the object. In some embodiments, the processing device 140 may control the first rotatable part of the image-guided radiation therapy system to rotate independently (with the second rotatable part remaining stationary), enabling the first detection assembly to receive the photon signal generated by the radioactive isotope within the object, thereby generating the first image of the target region. In some embodiments, the processing device 140 may control the first rotatable part of the image-guided radiation therapy system to remain stationary, allowing the first detection assembly to receive the photon signal generated by the radioactive isotope within the object, thereby generating the first image of the target region. In some embodiments, the processing device 140 may use a reconstruction algorithm to generate the second image. For example, the reconstruction algorithm may include an iterative reconstruction algorithm (e.g., a statistical reconstruction algorithm), a Fourier slice theorem algorithm, a filtered backprojection algorithm, a fan beam reconstruction algorithm, an analytical reconstruction algorithm, or any combination thereof.

In operation 1130, a radiation source is guided to emit a therapeutic beam toward the target region of the object based on the first image.

In some embodiments, during the radiation therapy process, the radiation source may emit a therapeutic beam toward the target region of the object based on the real-time acquired first image for radiation therapy. In some embodiments, the processing device 140 may adjust the therapeutic beam emitted by the radiation source in real time based on the first image. The therapeutic beam may be used for radiation therapy on the target region. In some embodiments, a movement of an organ (such as heart movement, respiratory movement, blood flow, muscle movement, pancreatic secretion activity, bladder movement, movement of the rectum and digestive system) within or near the target region of the object may cause a change in the position of the target region. During the radiation therapy process, the processing device 140 may monitor changes and/or locate the target region in real-time based on the first image to promptly adjust the therapeutic beam, ensuring the accuracy. For example, the processing device 140 may adjust a beam angle and/or a beam shape of the therapeutic beam by adjusting an upper treatment head and/or a collimation part based on a real-time obtained position, shape, etc., of the target region of the object in the first image. This ensures that the therapeutic beam targets at the target region while avoiding damage to surrounding organs (such as the liver, blood vessels, etc.) to better treat the target region without causing harm to other organ tissues.

In some embodiments, when movement or changes in the target region of the object are detected based on the first image, the radiation or the position of the object may be adjusted. For example, the medical device 110 may real-time adjust the upper treatment head to align the object with the moving or changing target region. As another example, the medical device 110 may suspend the emission of the therapeutic beam, adjust the position of the target region relative to the therapeutic beam by controlling the bed 115, and then resume the emission of the therapeutic beam after adjusting the radiation of the therapeutic beam or the position of the object. In some embodiments, when movement or changes in the target region are detected, the radiation source may terminate the emission of the therapeutic beam. In some embodiments, the processing device 140 may generate a notification based on a detected movement or change in the target region. For example, the processing device 140 may send the notification to the at least one terminal device 130 in the form of text, video, audio, etc., to alert the medical personnel of the movement or change in the target region.

It should be noted that the description of the image-guided radiation therapy method 1100 above is for illustration and explanation purposes only and does not limit the scope of the present disclosure. Those skilled in the art may make various modifications and changes to the image-guided radiation therapy method 1100 under the guidance of the present disclosure. For example, in operation 1110, the first detection assembly may be positioned at the second position. Accordingly, in operation 1120, at least one imaging source (e.g., a CT imaging source) may emit an imaging beam toward the target region of the object to obtain the second image, and in operation 1130, the radiation source may be guided to emit a therapeutic beam toward the target region of the object based on the second image in real-time. However, these modifications and changes are still within the scope of the present disclosure.

FIG. 12 is a flowchart illustrating an exemplary process of an image-guided radiation therapy method according to some other embodiments of the present disclosure.

In some embodiments, an image-guided radiation therapy method 1200 for an image-guided radiation therapy system (e.g., the image-guided radiation therapy systems 200 to 600) may be executed by a processing device (e.g., the processor 810 in FIG. 8, the processing device 140 in FIG. 1) or the image-guided radiation therapy system 1000. For example, the image-guided radiation therapy method 1200 may be stored in a storage device (e.g., the storage device 150, the memory 820, the storage 990) in the form of a program or instruction, and when the processing device 140 executes the program or instruction, the method 1200 may be implemented. As shown in FIG. 12, in some embodiments, before radiation therapy, the processing device 140 may cause an imaging source to emit an imaging beam toward an object to generate a second image of a target region of the object, so as to determine a position of the target region and/or adjust a treatment plan based on the second image.

In operation 1210, a first detection assembly may be positioned at a second position to obtain the second image of the target region.

In some embodiments, processing device 140 may send a control instruction to the image-guided radiation therapy system (e.g., the image-guided radiation therapy systems 200 to 600) to position the first detection assembly at the second position. For example, a control part of the medical device 110 may control a driving mechanism to drive a motion part to move a preset distance along a guide rail in a straight line based on the received control instruction, so as to switch the first detection assembly from a first position to the second position (e.g., the position of the first detection assembly 210 in FIG. 7(b)).

In some embodiments, after placing the object within a bore (e.g., the bore 116) of the image-guided radiation therapy system, the second image of the object may be generated by making a first rotatable part of the image-guided radiation therapy system (e.g., image-guided radiation therapy systems 200 to 600) to rotate independently with respect to a second rotatable part, thereby making the imaging source (e.g., the CT tube 251 or the DR tube 252) to emit an imaging beam toward the object. In some embodiments, the second image of the object may be generated by synchronously rotating the first rotatable part and the second rotatable part of the image-guided radiation therapy system (i.e., the first rotatable part and the second rotatable part are relatively stationary), making the imaging source (e.g., CT tube 251 or DR tube 252) to emit an imaging beam toward the object. In some embodiments, the second image of the object may be generated by keeping the first rotatable part of the image-guided radiation therapy system stationary, making the imaging source (e.g., CT tube 251 or DR tubes 252) emit an imaging beam toward the object. In some embodiments, a reconstruction algorithm may be used to generate the second image of the target region.

In some embodiments, the second image may include the target region of the object. For example, the CT tube 251 may emit an imaging beam toward the target region of the object, and the CT detector 253 may receive the entire or partial imaging beam to generate the second image of the target region. In some embodiments, the target region may be determined based on the second image of the object. For example, the processing device 140 may determine a lesion region of the object based on the second image, and determine the lesion region as the target region.

In operation 1220, the target region may be located and/or the treatment plan may be determined based on the second image.

In some embodiments, the treatment plan for the object may be determined based on the second image. In some embodiments, an adjustment to the treatment plan for the object may be made based on position information of the target region obtained from the second image. For example, the processing device 140 may register the second image of the object with a planned image (e.g., the second image taken during a previous session) to determine the position information of the target region. As another example, the processing device 140 may determine whether to adjust the treatment plan for the object based on the second image. In some embodiments, basic information about the object, such as body shape, weight, size, or position of the lesion to be treated, may differ from information used in planning the treatment plan. Therefore, adjustments to the treatment plan may be necessary. In some embodiments, the processing device 140 may determine the latest basic information of the object based on the second image and adjust the treatment plan accordingly.

In some embodiments, a therapeutic beam and/or the target region may be adjusted based on the position information and/or the treatment plan for the target region of the object. For example, the bed 115 may be moved based on the determined position information of the target region, placing the target region of the object in a center of a rotation plane of a treatment assembly in the medical device 110, allowing the therapeutic beam (e.g., the radiation source 220) to target at the target region. As another example, a parameter of the treatment assembly in the medical device 110 may be adjusted based on an adjusted treatment plan such that the therapeutic beam targets the target region.

It should be noted that the description of the image-guided radiation therapy method 1200 above is for illustration and explanation purposes only and does not limit the scope of the present disclosure. Those skilled in the art may make various modifications and changes to the image-guided radiation therapy method 1200 under the guidance of the present disclosure. For example, in operation 1210, the first detection assembly may be positioned at the first position. Accordingly, in operation 1220, the first detection assembly may obtain a first image, and in operation 1230, the target region may be located and/or the treatment plan determined based on the first image. However, these modifications and changes are still within the scope of the present disclosure.

FIG. 13 is a flowchart illustrating an exemplary process of an image-guided radiation therapy method according to some other embodiments of the present disclosure.

In some embodiments, an image-guided radiation therapy method 1300 for an image-guided radiation therapy system (e.g., the image-guided radiation therapy systems 200 to 600) may be executed by the processing device 140 (e.g., the processor 810 in FIG. 8, the processing device 140 in FIG. 1) or the image-guided radiation therapy system 1000. For example, the method 1300 may be stored in a storage device (e.g., the storage device 150, the memory 820, the storage 990) in the form of a program or instruction, and when the processing device 140 executes the program or instruction, the method 1300 may be implemented. As shown in FIG. 13, in some embodiments, during a radiation therapy process, a first detection assembly of the image-guided radiation therapy system (e.g., the image-guided radiation therapy systems 200 to 600) may be positioned at a second position to perform routine radiation therapy on a target region of an object.

In operation 1310, the first detection assembly may be positioned at the second position.

In some embodiments, the processing device 140 may send a control instruction to the image-guided radiation therapy system (e.g., the image-guided radiation therapy systems 200 to 600) to position the first detection assembly at the second position. For example, after the locking part 270 is controlled to lock the first rotatable part 230 and the second rotatable part 240, a control part of the medical device 110 may, based on the control instruction, drive a motion part of the first detection assembly 210 to move along a guide rail on the first rotatable part 230 by a preset distance, such that the first detection assembly 210 is positioned at the second position.

In operation 1320, a therapeutic beam may be emitted toward the target region of the object by making the second rotatable part rotate.

In some embodiments, the first rotatable part and the second rotatable part may be made to rotate synchronously based on a treatment plan to emit the therapeutic beam toward the target region of the object. In some embodiments, the second rotatable part may be made to rotate independently with respect to the first rotatable part to emit the therapeutic beam toward the target region of the object.

In some embodiments, during the radiation therapy of the target region, the processing device 140 may automatically generate and/or analyze an image to record the radiation therapy, monitor a position of the target region, assess a change in the position of the target region, and/or determine a plan for further radiation therapy (e.g., continuing the radiation therapy as planned, continuing the radiation therapy with an adjusted plan, terminating the radiation therapy, etc.). In some embodiments, a user input (e.g., from a doctor) may be utilized to semi-automatically perform the monitoring, assessment, and/or adjustment. For example, the processing device 140 may send one or more images (e.g., the first image, the second image) to be displayed on the at least one terminal device 130, allowing the user to analyze the image and provide one or more instructions on how to proceed with the treatment plan (e.g., continue the radiation therapy as planned, continue the radiation therapy with adjusted plans, terminate the radiation therapy, etc.).

It should be noted that the description of the image-guided radiation therapy method 1300 above is only for examples and illustrations and does not limit the scope of the present disclosure. For those skilled in the art, various modifications and changes may be made to the image-guided radiation therapy method 1300 under the guidance of the present disclosure. For example, the methods 1200 and 1100 may be integrated and executed together. The processing device 140 may locate the target region of the object and/or adjust the treatment plan based on the second image, accurately position the object, position the first detection assembly at the first position, generate the first image of the object, and perform real-time tracking and positional guidance therapy based on the first image. As another example, the methods 1200 and 1300 may be integrated. The processing device 140 may locate the target region of the object and/or adjust the treatment plan based on the second image, accurately position the object, further position the first detection assembly at the second position, and emit the therapeutic beam toward the target region of the object by moving a treatment head, achieving routine radiation therapy. However, these modifications and changes are still within the scope of the present disclosure.

The beneficial effects of the embodiments described in the present disclosure may include, but are not limited to: (1) by integrating the first detection assembly into the image-guided radiation therapy system and making the first detection assembly isocenteric with the radiation source, functions of PET bio-guided radiotherapy are realized, thereby improving treatment effectiveness and efficiency; (2) the first detection assembly may move between the first and second positions, and positioned at the second position during CT/DR imaging, thereby avoiding interference with CT/DR imaging and ensuring CT/DR imaging speed and image quality; (3) during the image-guided radiation therapy, the first detection assembly may be positioned at the first or second position, achieving functions of PET bio-guided radiotherapy and supporting routine radiation therapy, enhancing convenience and treatment efficiency; (4) by integrating the PET detector, the CT/DR imaging assembly, and the treatment assembly, the entire radiation therapy system becomes more compact while achieving multifunctional radiation therapy, reducing space requirements for a treatment room; (5) placing the imaging source in the first detection assembly ensures the image quality of both CT/DR imaging and PET imaging during radiation therapy; (6) setting the first detection assembly and imaging source side by side can optimize the image quality of PET imaging while avoiding the impact of PET imaging on CT/DR imaging; (7) by moving the PET detector out of the isocenter during routine radiation therapy, the PET detector is protected from unnecessary radiation damage from the therapeutic beam.

It should be noted that different embodiments may have different beneficial effects. In different embodiments, the possible beneficial effects may be any combination of the above, or any other possible beneficial effects.

The basic concepts have been described above, and it is apparent that to a person skilled in the art, the above detailed disclosure is intended as an example only and does not constitute a limitation of the present disclosure. Although not expressly stated herein, various modifications, improvements, and amendments may be made to the present disclosure by those skilled in the art. Such modifications, improvements, and amendments are suggested in the present disclosure, so such modifications, improvements, and amendments remain within the spirit and scope of the exemplary embodiments of the present disclosure.

Also, the present disclosure uses specific words to describe the embodiments of the present disclosure. For example, "an embodiment", "one embodiment", and/or "some embodiments" are meant to refer to a certain feature, structure, or characteristic associated with at least one embodiment of the present disclosure. Accordingly, it should be emphasized and noted that "an embodiment" or "one embodiment" or "an alternative embodiment" mentioned two or more times in different places in the present disclosure do not necessarily refer to the same embodiment. Furthermore, certain features, structures, or characteristics in one or more embodiments of the present disclosure may be suitably combined.

Furthermore, unless expressly stated in the claims, the order of processing elements and sequences, the use of numerical letters, or the use of other names described herein are not intended to limit the order of the processes and methods of the present disclosure. Although a number of embodiments of the present disclosure currently considered useful are discussed in the above disclosure by way of various examples, it should be understood that such details serve illustrative purposes only, and that additional claims are not limited to the disclosed embodiments; rather, the claims are intended to cover all amendments and equivalent combinations that are consistent with the substance and scope of the embodiments of the present disclosure. For example, although the implementation of various components described above may be embodied in a hardware device, it may also be implemented as a software only solution, e.g., an installation on an existing server or mobile device.

## Claims

1. An image-guided radiation therapy system (100, 200, 300, 400, 500, 600, 700), comprising:
a first detection assembly (210) configured to determine a first image of a target region of an object by receiving a photon signal generated by a radioactive isotope within the object;
at least one imaging source configured to determine a second image of the target region by emitting an imaging beam to the target region of the object; and
a radiation source (220) configured to emit a therapeutic beam to the target region of the object, wherein,
the first detection assembly (210) and the at least one imaging source are mounted on a first rotatable part (230), the radiation source (220) is mounted on a second rotatable part (240), and the first detection assembly (210) is movable relative to the first rotatable part (230).

2. The image-guided radiation therapy system (100, 200, 300, 400, 500, 600, 700) of claim 1, wherein the first detection assembly (210) is movable along a rotational axis direction of the first rotatable part (230).

3. The image-guided radiation therapy system (100, 200, 300, 400, 500, 600, 700) of claim 2, wherein the first detection assembly (210) is movable between a first position and a second position along the rotational axis direction of the first rotatable part (230).

4. The image-guided radiation therapy system (100, 200, 300, 400, 500, 600, 700) of claim 3, wherein the first detection assembly (210) switches between the first position and the second position by moving a preset distance, the preset distance is determined based on a thickness of the first detection assembly (210) along the rotational axis direction.

5. The image-guided radiation therapy system (100, 200, 300, 400, 500, 600, 700) of claim 3 or 4, wherein the at least one imaging source includes a computed tomography (CT) imaging source and/or a digital radiography (DR) imaging source.

6. The image-guided radiation therapy system (100, 200, 300, 400, 500, 600, 700) of any one of claims 3-5, further comprising:
a second detection assembly configured to determine a second image of the target region of the object by detecting at least a portion of an imaging beam emitted to the object by the at least one imaging source, wherein the first detection assembly (210) includes a PET detector or a SPECT detector, the second detection assembly includes at least one of a therapeutic beam detector (225), a CT detector (253), or a DR detector (254).

7. The image-guided radiation therapy system (100, 200, 300, 400, 500, 600, 700) of claim 5 or 6, further comprising:
a collimation part (223), wherein the second detection assembly includes the CT detector (253) and the therapeutic beam detector (225), the at least one imaging source includes a CT tube (251), when viewed from a side of the image-guided radiation therapy system, the first detection assembly (210) is at the first position, the first detection assembly (210), the radiation source (220), the collimation part (223), the therapeutic beam detector (225), the CT tube (251), and the CT detector (253) are located on a same line.

8. The image-guided radiation therapy system (100, 200, 300, 400, 500, 600, 700) of claim 7, wherein when the first detection assembly (210) is at the second position, the radiation source (220), the collimation part (223), the therapeutic beam detector (225), the CT tube (251), and the CT detector (253) are located on a first straight line, while the first detection assembly (210) is on a second straight line.

9. The image-guided radiation therapy system (100, 200, 300, 400, 500, 600, 700) of claim 5 or 6, further comprising:
a collimation part (223), wherein the second detection assembly includes the DR detector (254) and the therapeutic beam detector (225), the at least one imaging source includes a DR tube (252), when viewed from a side of the image-guided radiation therapy system, the first detection assembly (210) is at the first position, the first detection assembly (210), the radiation source (220), the collimation part (223), the therapeutic beam detector (225), the DR tube (252), and the DR detector (254) are located on a same line; and
when the first detection assembly (210) is at the second position, the radiation source (220), the collimation part (223), the therapeutic beam detector (225), the DR tube (252), and the DR detector (254) are located on a first straight line, while the first detection assembly (210) is on a second straight line.

10. The image-guided radiation therapy system (100, 200, 300, 400, 500, 600, 700) of any one of claims 3-9, wherein the first rotatable part (230) includes a guide rail, the first detection assembly (210) includes a motion part that matches the guide rail and a corresponding driving mechanism, and the driving mechanism is configured to drive the motion part on the first detection assembly (210) to move along the guide rail by a preset distance, allowing the first detection assembly (210) to switch between the first position and the second position.

11. The image-guided radiation therapy system (100, 200, 300, 400, 500, 600, 700) of any one of claims 3-6, wherein when the first detection assembly (210) is at the first position or the second position, the at least one imaging source, the first detection assembly (210), and the radiation source (220) are coplanar in a longitudinal direction.

12. The image-guided radiation therapy system (100, 200, 300, 400, 500, 600, 700) of any one of claims 3-5, wherein the first detection assembly (210) includes at least two sets of detectors, the at least two sets of detectors are arranged in opposition, and when the at least two sets of detectors are at the first position, the at least one imaging source is obscured by at least one detector of the at least two sets of detectors.

13. The image-guided radiation therapy system (100, 200, 300, 400, 500, 600, 700) of any one of claims 2-12, wherein the first detection assembly (210) is rotatable relative to the first rotatable part (230).

14. The image-guided radiation therapy system (100, 200, 300, 400, 500, 600, 700) of any one of claims 1-13, wherein the first rotatable part (230) rotates on a first rotation plane, the second rotatable part (240) rotates on a second rotation plane, and the first rotation plane and the second rotation plane are different.

15. The image-guided radiation therapy system (100, 200, 300, 400, 500, 600, 700) of any one of claims 1-14, further comprising: a locking part (270) configured to lock the first rotatable part (230) and second rotatable part (240), such that the first rotatable part (230) and second rotatable part (240) rotate synchronously.
